# EUROPEAN PATENT SPECIFICATION

(11) **EP 4 232 189 B1**
(45) Date of publication and mention of the grant of the patent: **15.05.2024**
(21) Application number: 21791421.7
(22) Date of filing: 21.10.2021
(51) Int. Cl.: B01J 19/18, B01D 61/04, C07K 1/34, B01J 4/00, B01J 4/02, B01J 19/00

(54) **NANOFILTRATION ASSISTED METHODS OF CHEMICAL SYNTHESIS**
NANOFILTRATIONSGESTÜTZTE VERFAHREN ZUR CHEMISCHEN SYNTHESE
PROCÉDÉS DE SYNTHÈSE CHIMIQUE ASSISTÉS PAR NANOFILTRATION

(30) Priority: 21.10.2020 EP 20203008
(43) Date of publication of application: 30.08.2023
(73) Proprietor: Bachem AG, 4416 Bubendorf (CH)
(72) Inventor: MARCHETTI, Patrizia, 5000 Aarau (CH); SAMSON, Daniel, 4051 Basel (CH); JAGIELSKI, Jakub, 4310 Rheinfelden (CH)
(74) Representative: Jacobi, Markus Alexander
(86) International application number: PCT/EP2021/079185
(87) International publication number: WO 2022/084441

(56) References cited:
- WO-A1-2016/042066
- WO-A1-2018/207904
- US-A- 6 001 966
- ORMEROD DOMINIC ET AL: "Cyclic Peptide Formation in Reduced Solvent Volumes via In-Line Solvent Recycling by Organic Solvent Nanofiltration", ORGANIC PROCESS RESEARCH & DEVELOPMENT, vol. 19, no. 7, 17 July 2015 (2015-07-17), pages 841-848, XP055782152, US ISSN: 1083-6160, DOI: 10.1021/acs.oprd.5b00103 cited in the application

## Description

The present invention generally relates to the field of organic synthesis at an industrial or laboratory scale. Improved methods of synthesis, in particular for reactions where one reactant is to be used in large excess over the other, are disclosed. Such reactions may occur in the preparation of cyclic peptides, cyclic oligonucleotides or oligonucleotide analogs, and conjugates of peptides, proteins, oligonucleotides, or oligonucleotide analogs with a carrier compound. The present invention is directed to methods of effectively preparing such molecules, which are of considerable commercial importance as active pharmaceutical ingredients.

In order to suppress side reactions, some reactions need to be carried out in a very dilute solution of a first reactant. This may be necessary, e.g., to suppress oligomerization, which may occur during the circularization of linear peptide precursors, or due to limited solubility of the first reactant in a required reaction medium. However, if such a reaction requires additionally the presence of a second reactant in large excess over the first reactant, it may become challenging to conduct the process in a way, which is economically viable, in a continuous stirred tank reactor or a continuous flow reactor.

Membrane filtration has been used to reduce consumption of solvents in the synthesis of disulfide-bonded peptides (Ormerod et al, Org. Process Res. Dev. 2015, 19, 841-848; WO 2016/042066; WO 2013/156600; Marchetti et al., Sustainable nanotechnology and the environment: Advances and achievements, 2013, Vol. 1124, pages: 121-150).

There is still a need in the art for a reliable, scalable, reproducible, and cost-effective method for carrying out synthesis reactions, where one reactant is used in excess over the other. The present inventors developed a method and apparatus allowing carrying out such reactions by combining a pseudo-high dilution approach with a membrane filtration, to control reaction conditions in the reaction vessel and optimize the addition and consumption of reagents.

In general, several definitions are used throughout the present application:
Unless otherwise stated, the term "about" means deviations by plus or minus 10% of the numeric value given. Unless otherwise stated, the expression "essentially" is used herein to reflect minor fluctuations, which may occur in a real-world setup. Unless otherwise stated, pH values are indicated for the temperature at which the respective aqueous solution is to be used.

Amino acids will be referred to interchangeably by either their full name (exemplified: alanine), 3-letter code according to WIPO Standard ST. 25 (e.g. Ala), or 1-letter code (e.g. A). As far as the enantiomeric form is not expressly specified, L-amino acids are in general referred to. It should be noted, however, that the present invention can likewise be put to practice using D-amino acids and other stereoisomers.

As used herein, the term "peptide" and "polypeptide" may be understood interchangeably. Unless indicated otherwise, peptide sequences are indicated herein starting with the N-terminus (left) and ending with the C-terminus (right). A peptide is characterized by the presence of at least one peptide bond (-CO-NH-) between at least two amino acids, i.e. moieties comprising a carboxyl (-COOH) and a primary or secondary amino group (-NHR). It is therefore to be understood that the term "peptide" is not limited to peptides build from naturally occurring amino acids. It also includes, inter alia, peptide derivatives, peptides comprising non-natural amino acids, peptides comprising D-amino acids, and peptides comprising covalently or non-covalently attached linkers, protecting groups, dyes, or other moieties. Peptides may be built of between 2 and 50 amino acids.

As used herein, the expression "oligonucleotide" is used in a most general way to relate to any oligomers comprising at least two nucleoside units linked by a phosphodiester bond or by an analogous structure. Nucleoside units are glycosylamines comprising a sugar moiety, commonly a ribose moiety, and a nucleobase. As used herein, the expression nucleoside unit encompasses the naturally occurring nucleosides as well as artificial compounds. The latter may carry substituents in the sugar moiety, e.g. -F, -OMe (-O-CH3), or -methoxyethyl (-O-CH2-CH2-O-CH3, aka. MOE, -O-methoxyethyl substituents, and/or may exhibit artificial analogs of nucleobases or abasic sites, and/or modifications of phosphate backbone, as are present, e.g., in phosphorothioates, phosphorodithioates, diastereomerically pure phosphorothioates, or sulfonyl phosphoramidates. Further non-limiting examples of oligonucleotides are locked nucleic acids (LNA), amino LNA, constrained ethyl nucleic acid analogs (cET), bridged nucleic acids (BNA), tricycloDNA, unlocked nucleic acids (UNA), and phosphoramidite morpholino oligonucleotides (PMO). As used herein, the term "oligonucleotide" and "polynucleotide" may be understood interchangeably. The expression "oligonucleotide" comprises in particular DNA, RNA, iRNA, dsRNA, and oligonucleotide phosphorothioates, as well as derivatives and analogs thereof.

The term "derivative" or "derivatives" as used herein refers to a compound, which can be obtained from a first compound by a chemical reaction. As a result, a derivative may differ from the first compound by the presence or absence of substituents. For example, amino acid derivatives for use in SPPS usually differ from the amino acid they are derived from at least by the presence of an amino protecting group.

The term "protecting group" as used herein may be understood in the broadest sense as a group which is introduced into a molecule by chemical modification of a functional group to block said group from reaction in subsequent process steps, e.g. to prevent side reactions of the amino acid side chains. Examples of amino protecting groups are the Boc and Fmoc groups, examples of carboxylic acid protecting groups are unreactive esters such as methyl esters, benzyl esters, or tert. butyl esters. Examples of sulfhydryl protecting groups, comprise, e.g. Acm, Phacm, Trt, Mtt, Mmt, Dpm, Bzl, tBu, and StBu protecting groups.

For the purpose of the present application, the terms "raw" and "crude" are used interchangeably to designate preparations of a compound, e.g. a peptide, which are essentially a direct product of synthesis and isolation processes and have not yet been submitted to specific purification steps. Chemical synthesis usually yields crude peptide preparations having a purity of around 40 to 80%.

In the context of the present invention, the term "purified" is used to designate compositions, e.g. peptide compositions, which have been subjected to specific purification steps, e.g. to preparative chromatography. Such compositions may be highly or partially purified and may have purities of up to 100%. It should however be understood that the present invention may be advantageously applied to crude, partially purified, and purified peptide compositions.

The present invention provides, *inter alia,* a method for reacting a first and a second compound, thereby forming a third compound, the method comprising the following steps:
a) Providing a first solution S1 comprising the first compound and a second solution S2 comprising the second compound;
b) Providing, inside a reaction vessel, a solution S3 comprising the second compound in a liquid reaction medium, which comprises an organic solvent; and
c) Concomitantly adding the solutions S1 and S2 over an extended period of time into the solution S3 while mixing the content of the reaction vessel, thereby forming a reaction mixture comprising the third compound;
wherein:
d) The second compound is in excess over the first compound inside the reaction vessel during step c); and
e) During step c), the content of the reaction vessel is subjected to membrane filtration so as to essentially retain the first compound, the second compound, and the third compound inside the reaction vessel.

The first and the second compound may be organic compounds, and at least one of the first and the second compound may comprise a carboxyl group or a hydroxyl group. The first and the second compound may be different from each other. At least one of the first and second compound may be a polymer. At least one of the first and the second compound may be a peptide or a peptide derivative. At least one of the first and the second compound may be an oligonucleotide or an oligonucleotide analog. At least one of the first and the second compound may be polyethylene glycol or a derivative of polyethylene glycol. At least one of the first and the second compound may be a dye. In some embodiments of the present invention, the first compound may be a peptide and the second compound may be an activating reagent. For example, the first compound may be a peptide and the second compound may be a carboxyl group activating reagent. In other embodiments of the present invention, the first compound may be a polymer and the second reagent may be an activating reagent. For example, the first compound may be a polyethylene glycol (PEG) or a PEG derivative and the second reagent may be an activating reagent. In other embodiments of the present invention, the first compound may be a polymer and the second reagent may be hydroxyl group activating reagent. For example, the first compound may be a polyethylene glycol (PEG) or a PEG derivative and the second reagent may be hydroxyl group activating reagent. In yet other embodiments, the first compound may be a polymer and the second compound may be a peptide. For example, the first compound may be an activated PEG or a PEG derivative and the second compound may be a peptide. In yet other embodiments, the first compound may be a peptide and the second compound may be a polymer. For example, the first compound may be a peptide and the second compound may be an activated PEG or a PEG derivative. In yet other embodiments, the first compound may be a peptide or a protein and the second compound may be a dye. In yet other embodiments, the first compound may be a polymer and the second compound may be a protein. For example, the first compound may be an activated PEG or a PEG derivative and the second compound may be a protein. In yet other embodiments, the first compound may be a protein and the second compound may be a polymer. For example, the first compound may be a protein and the second compound may be an activated PEG or a PEG derivative. In yet other embodiments, the first compound may be a dye and the second compound may be a protein or a peptide.

As used herein, the expression "activating reagent" is used to refer to a reagent, which reacts with a functional group of another compound, thereby facilitating subsequent reactions. Carboxyl group activating reagents comprise, among many others, 6-Chloro-benzotriazole-1-yloxy-tris-pyrrolidinophosphonium hexafluorophosphate (PyClock), Benzotriazol-1-yl-oxy-tris-pyrrolidinophosphonium hexafluorophosphate (PyBOP), 1-Cyano-2-ethoxy-2-oxoethylideneaminooxy-tris-pyrrolidino-phosphonium hexafluorophosphate (PyOxim), (7-Azabenzotriazol-1-yloxy)trispyrrolidinophosphonium hexafluorophosphate (PyAOP), Bromo-tris-pyrrolidino-phosphonium hexafluorophosphate (PyBroP), Benzotriazole-1-yl-oxytris-(dimethylamino)-phosphoniumhexafluorophosphate (BOP), 1-[(1-(Cyano-2-ethoxy-2-oxoethylideneam inooxy)-dimethylam ino-morpholino)] uronium hexafluorophosphate (COMU), 2-(6-Chloro-1-H-benzotriazole-1-yl)-1,1,3,3-tetramethylaminium hexafluorophosphate (HCTU), 1-[Bis(dimethylamino)methylene]-1H-1,2,3-triazolo[4,5-b]pyridinium 3-oxide hexafluorophosphate (HATU), 2-(1H-Benzotriazole-1-yl)-1,1,3,3-tetramethylaminium hexafluorophosphate (HBTU), O-[(Ethoxycarbonyl)cyanomethylenamino]-N,N,N',N'-tetramethyluronium tetrafluoroborate (TOTU), O-(Benzotriazol-1-yl)-N,N,N',N'-tetramethyluranium tetrafluoroborate (TBTU), O-(7-Azabenzotriazole-1-yl)-N,N,N',N'-tetramethyluronium tetrafluoroborate (TATU), N,N'-Dicyclohexylcarbodiimide (DCC), *N,N'*-Diisopropylcarbodiimide (DIC) and Ethyl-(*N',N'-*dimethylamino)propylcarbodiimide hydrochloride (EDC HCl); they are sometimes referred to as coupling reagents. An example of a hydroxyl group activating reagent is a *N,N'*-Disuccinimidyl carbonate (DSC). Reaction with alcohols results in the formation of a succinimidylcarbonate, which reacts with amines to form a carmabate. This chemistry may be employed, for example, to couple PEG or a PEG derivative to the amino groups of peptides and proteins.

As used herein, S1, S2, and S3 are reference signs, which have no meaning beyond facilitating reading of the present document. The solution S1 is the solution comprising the first compound prepared in step a). The solution S2 is the solution comprising the second compound prepared in step a). The solution S3 is the solution comprising the second compound inside a liquid reaction medium prepared in step b). The solutions S1 and S2 may be solutions of said compounds in an organic solvent or in a mixture of solvents.

The terms "providing a solution S1 comprising the first compound", "providing a solution S2 comprising the second compound" and "providing a solution S3 of the second compound in a liquid reaction medium" may be understood in the broadest sense as obtaining any solution containing the compound of interest. Said compounds may be provided by any means known in the art. Exemplarily, a peptide or a partially protected peptide may be obtained from Solid Phase Peptide Synthesis (SPPS), e.g. Fmoc-SPPS, or Liquid Phase Peptide Synthesis (LPPS) or a combination thereof. After SPPS, the peptide may be cleaved from the resin using a cleavage cocktail comprising an acid such as TFA and one or more scavengers such as water, triisopropylsilane (TIPS), dithiothreitol (DTT), dithioerythriol (DTE), anisole, thioanisole or 1 ,2-ethanedithiol (EDT). Depending on the resin used for SPPS, cleavage may be performed without affecting side-chain protecting groups. The peptide may then be precipitated from the cleavage cocktail using an organic anti-solvent such as, e.g., diethyl ether, diisopropyl ether, methyl tert. butyl ether (MTBE), or mixtures of the aforementioned ethers with, e.g., acetonitrile or hexane. Alternatively, the plain polypeptide strand may also be obtained from a biotechnological method and optionally be modified by chemical/synthetic means subsequently. As a further example, many coupling reagents, PEG, and PEG derivatives are commercially available.

It will be understood by a person skilled in the art that the first and second compounds contained in the solutions provided in steps a) and b) may optionally bear any counter ions known in the art, such as anions or cations, such as e.g., chloride ions, acetate ions, carbonate ions, hydrocarbonate ions, sodium ions, potassium ions, magnesium ions , bromide ions, perchlorate ions, ammonium ions, phosphate ions, or sulfate ions, any ions of the cleavage solution employed in solid phase synthesis (e.g., TFA ions) and/or cations or anions of residuals of protecting groups. Further, in cases where the first or the second compound is a peptide, such a peptide may optionally be covalently or non-covalently associated to traces of one or more scavengers, such as, e.g., triisopropylsilane (TIPS), dithiothreitol (DTT), dithioerythriol (DTE), anisole, thioanisole or 1,2-ethanedithiol (EDT) or may contain traces of one or more anti-solvents such as diisopropyl ether, diethyl ether, acetonitrile, hexane, or methyl tert. butyl ether. The solution of step a) may comprise one or more of the above-mentioned ions and compounds, as well as any adducts, polymers, or other products formed by chemical reactions of the above-mentioned compounds. In one embodiment, the solution of step a) may comprise a crude or raw peptide, which may have a purity of 30-90%, e.g. of above 30%, 40%, 50%, 60%, 70%, 80% or 90%. In some embodiments, a raw peptide having a purity of 40-80% is used. In one embodiment, the crude peptide composition has been obtained by precipitating the peptide out of a cleavage cocktail following SPPS. In one preferred embodiment, the solution of step a) may comprise a partially protected crude peptide, i.e. a peptide composition, where at least some protecting groups have not been removed and which has not been subjected to specific purification steps such as preparative chromatography. In other embodiments, the solution of step b) may comprise a partially or highly purified peptide.

The solutions S1 and S2 of step a) may preferably comprise one or more organic solvents such as DMF, DCM, methyl tert-butyl ether, methanol, isopropyl alcohol, acetonitrile, or others. For example, the solutions of step a) may be obtained by dissolving or diluting a crude or purified peptide preparation in an organic solvent or in a mixture of organic solvents such as DMF, methanol, isopropyl alcohol, acetonitrile, or others. As a further example, the solutions of step a) may comprise PEG in an organic solvent such as DMF, dioxane or acetonitrile. Preferably, the solutions of step a) are prepared so as to minimize the content in components other than the first compound, which may react with any of the compounds provided inside the reaction vessel in step c). Preferably, the solutions of step a) are miscible with the contents of the reaction vessel provided in step b), so that mixing both solutions yields a homogenous solution. Preferably, the solutions S1 and S2 are a solution of the respective compound in the liquid reaction medium or in a solvent miscible with the liquid reaction medium.

As used herein, the expression "liquid reaction medium" may refer to the liquid composition, in which the first and the second compound react with each other. The liquid reaction medium may comprise one or more solvents and further compounds such as, e.g., proton donors and proton acceptors. By contrast, the expression "reaction mixture" may be used to refer to the entire solution contained inside the reaction vessel during step c). In other words, the reaction mixture comprises the liquid reaction medium plus the first compound, the second compound, any additional solvents introduced with the solutions S1 and S2, and any reaction products, in particular the third compound, formed between these compounds. The person skilled in the art will understand that the composition of the liquid reaction medium of step b) is influenced by the properties of the first and the second compound, as well as by the properties of the third compound and other products formed by their chemical reaction. Further, the skilled person is aware that the liquid reaction medium may facilitate the desired chemical reaction between the first and the second compound, e.g. by having a stabilizing effect on a transition state occurring during said reaction. Preferably, the reaction medium allows dissolving the educts and products of the reaction at the concentrations occurring inside the reaction vessel. It is preferred that the reaction medium does not react with the first, second, or third compound. The reaction medium may be essentially inert towards the first, second, or third compound. Bases such as DIPEA, Dimethylaminopyridine (DMAP) or proton donors such as organic or inorganic acids may be included in the reaction medium so as to generate a suitable environment for the desired chemical reaction to occur. The reaction medium may comprise or essentially consist of one or more organic solvents such as DMF, DCM, methyl tert-butyl ether, methanol, ethanol, isopropyl alcohol, acetonitrile, or others. The skilled artisan will routinely select a suitable liquid reaction medium.

In in step c) of the method according to the present invention, solutions S1 and S2 are added concomitantly into the solution S3 contained inside the reaction vessel over an extended period of time. As used herein, the expression "over an extended period of time" means that the solutions S1 and S2 are added gradually, e.g. by a continuous flow of liquid or by stepwise addition of aliquots, into the reaction medium. The time needed may depend on the volumes and concentrations of the solutions used, on the speed of the chemical reaction between the first and the second compound, and on the desired amount of the third compound to be produced.

Without wishing to be bound by theory, it is believed that during step c), the addition of the first compound may be balanced at least in part by its consumption due to chemical reaction with the second compound, thereby limiting the concentration of the first compound inside the reaction vessel. As used herein, the expression "concentration inside the reaction vessel" may be synonymous with the expression "concentration inside the liquid contained inside the reaction vessel". It may be used to refer to the concentration of a compound inside a liquid volume, which does result or would result from the instantaneous and homogenous distribution of said substance inside the volume. Concentration may be indicated in gram per liter or mole per liter.

In some embodiments, the concentration of the first compound inside the reaction vessel may be close to zero, i.e. below the detection limit, during step c). In some embodiments, the concentration of the first compound inside the reaction vessel is below 10 mmol/l (mM), below 1 mM, below 0.1 mM, below 0.01 mM, below 1 micromol/l (µM), below 0.1 µM, or below 0.01 µM. The addition of the solution comprising the first compound into the reaction vessel may take at least 1, 2, 5, 10, 15, 20, 25, 30, 35, 40, 45, 50, 55, 60, 70, 80, or 90 minutes.

In a preferred embodiment, the rate of addition of the first compound is lower than or equal to the rate of consumption of the first compound by the chemical reaction between the first and the second compound over the duration of step c). In a preferred embodiment, the rate of addition of the first compound is lower than or equal to the rate of consumption of the first compound by the chemical reaction between the first and the second compound. The rates of addition and consumption may be expressed in molar amount of the first compound added or consumed per time, respectively.

Therefore, one embodiment of the invention relates to a method for reacting a first compound and a second compound, thereby forming a third compound, the method comprising the following steps:
a) Providing a first solution S1 comprising the first compound and a second solution S2 comprising the second compound;
b) Providing, inside a reaction vessel, a solution S3 comprising the second compound in a liquid reaction medium, which comprises an organic solvent; and
c) Concomitantly adding the solutions S1 and S2 into the solution S3 while mixing the content of the reaction vessel, thereby forming a reaction mixture comprising the third compound, wherein the rate of addition of the first compound is lower than or equal to the rate of consumption of the first compound by the chemical reaction between the first and the second compound;
further wherein:
d) The second compound is in excess over the first compound inside the reaction vessel during step c); and
e) During step c), the content of the reaction vessel is subjected to membrane filtration so as to essentially retain the first compound, the second compound, and the third compound inside the reaction vessel.

As used herein, the expression "concomitantly" may mean that the two solutions are added essentially at the same time or promptly after each other. This may be performed by allowing the solutions S1 and S2 to flow continuously into the reaction vessel at the same time. However, each of the flows of the solutions S1 and S2 may also be interrupted while the flow of the other solution continues during step c), or they may be dosed in alternating intervals of time during step c). As a further example, one of the solutions may be allowed to flow continuously into the reaction vessel, while the other solution may be dosed in stepwise fashion.

The first compound and the second compound may react spontaneously upon being exposed to each other inside the liquid reaction medium. The skilled person will routinely choose the chemical composition of the liquid reaction medium and control the physical parameters of the reaction medium inside the reaction vessel, e.g. temperature and pressure, so as to favor the desired reaction between the first and the second compound. As used herein, the expressions "react", "reaction" and the like are used to describe an interaction between two compounds, which leads to the breaking of chemical bonds and the making of new chemical bonds, thereby forming at least one, structurally different, third compound. The chemical bonds may be covalent bonds.

The addition of the solutions into the reaction vessel, for example the addition of the solutions S1 and S2 during step c), may be effected and controlled by any means known in the art. For instance, the liquid flow may be driven by gravity, by pressure applied to a feed tank comprising the solutions, or by a pump. The flow may be regulated by means of a valve and/or by regulating the action of the pump. In preferred embodiments, the flow of the solutions S1 and S2 during step c) of the present method is driven by pumps.

The person skilled in the art will understand that mixing of reaction vessel's contents may be achieved by any suitable means. For example, a stirrer comprising a rotating impeller may be used. Such an impeller may be a turbulent mixer causing axial, mixed, or radial flow of the liquid inside the reaction vessel. Known impellers include marine-type propellers, pitch-blade turbines, flat-blade turbines, and flat-blade paddles. The use of baffle blades may be helpful to improve mixing. Alternatively or in addition, mixing may be achieved by bubbling gas through the liquid. Alternatively or in addition, mixing may be achieved by liquid circulation, e.g. by means of a pumping circuit. A person skilled in the art will usually choose the mixing means so as to achieve efficient distribution of substances inside the reaction medium while avoiding foaming.

Preferably, the solution S2 is a solution of the second compound in the liquid reaction medium or in a solvent miscible with the liquid reaction medium. The skilled person will routinely adjust the concentration of the second compound in said solution so that it allows for convenient and precise dosing of the amounts of second compound needed. The concentration of the second compound inside the solution S2 may be higher than the concentration of the second compound inside the solution S3. The flow of said solution into the reaction vessel may be adjusted such that the consumption of the second compound by its reaction or reactions inside the reaction vessel may be balanced by the influx of the second compound.

Dosing the flow of the solutions S1 and S2 into the reaction vessel may be continuous and/or controlled by feedback loops. For example, the flow of the solution S1 and/or of the solution S2 may be controlled based on optical properties of the liquid contained in the reaction vessel. In some embodiments, the flow of the solution of step c) is controlled by a feedback signal generated by a monitoring system inside the reaction vessel. It is noted that the monitoring system may take the form of a dip probe or of a flow cell and may be installed inside the reaction vessel itself, or - in embodiments where the reaction vessel is part of a pumping circuit - inside the recirculation loop or in a bypass to that loop. For example, photometric measurements may provide a feedback signal regulating the flow of reagents into the reaction vessel. In some embodiments of the present invention, the flow of the solution of step a) into the reaction vessel is independently controlled and may be continuously adjusted.

As used herein, the expression "during step ..." may mean over at least 90% of the whole duration of said step. For instance, "during step c)" may mean over the duration of at least 90%, 91%, 92% 93%, 94%, 95%, 96%, 97%, 98%, or 99% of the time, during which the first solution S1 and the second solution S2 are added into the reaction vessel.

According to the inventive method, the second compound is in excess over the first compound inside the reaction vessel. This may be understood to mean that the equivalent concentration of the first compound is below the equivalent concentration of the second compound inside the reaction vessel. The equivalent concentration c_{eq}, also known as normality, may be defined as the molar concentration c of a compound divided by an equivalence factor f_{eq}. The equivalence factor f_{eq} may reflect the stoichiometry of the reaction between the first and the second compound. For example, if one molecule of the first compound reacts with two molecules of the second compound, the equivalence factor f_{eq} for the first compound will be 0.5 and the equivalence factor f_{eq} for the second compound will be 1. As the concentrations of the first and the second compound inside the reaction vessel may fluctuate over time during step c), it may be considered that the second compound is in excess over the first compound if the equivalent concentration c_{eq}, of the second compound inside the reaction vessel is higher than the equivalent concentration c_{eq}, of the first compound inside the reaction vessel in at least 90%, preferably 100% of the total time of step c) - The equivalent concentration c_{eq}, of the second compound inside the reaction vessel may be higher than the equivalent concentration c_{eq}, of the first compound inside the reaction vessel in more than 90%, 91%, 92%, 93%, 94%, 95%, 96%, 97%, 98%, or 99% the total time of step c). In some embodiments, the equivalent concentration c_{eq}, of the second compound inside the reaction vessel is higher than the equivalent concentration c_{eq}, of the first compound inside the reaction vessel by a factor of at least 1.5 in more than 90% of the total time of step c). In some embodiments, the equivalent concentration c_{eq}, of the second compound inside the reaction vessel is higher than the equivalent concentration c_{eq}, of the first compound inside the reaction vessel by a factor of at least 2 in more than 90% of the total time of step c). In some embodiments, the equivalent concentration c_{eq}, of the second compound inside the reaction vessel is higher than the equivalent concentration c_{eq}, of the first compound inside the reaction vessel by a factor of at least 2.5 in more than 90% of the total time of step c). In some embodiments, the equivalent concentration c_{eq}, of the second compound inside the reaction vessel is higher than the equivalent concentration c_{eq}, of the first compound inside the reaction vessel by a factor of at least 3 in more than 90% of the total time of step c). In some embodiments, the equivalent concentration c_{eq}, of the second compound inside the reaction vessel is higher than the equivalent concentration c_{eq}, of the first compound inside the reaction vessel by a factor of at least 100 in more than 90% of the total time of step c). In some embodiments, the equivalent concentration c_{eq}, of the second compound inside the reaction vessel is higher than the equivalent concentration c_{eq}, of the first compound inside the reaction vessel by a factor of at least 1.5 in more than 95% of the total time of step c). In some embodiments, the equivalent concentration c_{eq}, of the second compound inside the reaction vessel is higher than the equivalent concentration c_{eq}, of the first compound inside the reaction vessel by a factor of at least 2 in more than 95% of the total time of step c). In some embodiments, the equivalent concentration c_{eq}, of the second compound inside the reaction vessel is higher than the equivalent concentration c_{eq}, of the first compound inside the reaction vessel by a factor of at least 2.5 in more than 95% of the total time of step c). In some embodiments, the equivalent concentration c_{eq}, of the second compound inside the reaction vessel is higher than the equivalent concentration c_{eq}, of the first compound inside the reaction vessel by a factor of at least 3 in more than 95% of the total time of step c). In some embodiments, the equivalent concentration c_{eq}, of the second compound inside the reaction vessel is higher than the equivalent concentration c_{eq}, of the first compound inside the reaction vessel by a factor of at least 100 in more than 95% of the total time of step c). In some embodiments, the equivalent concentration c_{eq}, of the second compound inside the reaction vessel is higher than the equivalent concentration c_{eq}, of the first compound inside the reaction vessel by a factor of at least 1.1, 1.2, 1.5, 2, 2.5, 3, 4, 5, 6, 7, 8, 9, 10, 20, 30, 40, 50, 60, 70, 80, 90, 100, 200, 300, 400, 500, 600, 700, 800, 900, or 1000 during the entire time of step c). In some embodiments, the equivalent concentration c_{eq}, of the second compound inside the reaction vessel is higher than the equivalent concentration c_{eq}, of the first compound inside the reaction vessel by a factor of at least 1.1, 1.2, 1.5, 2, 2.5, 3, 4, 5, 6, 7, 8, 9, 10, 20, 30, 40, 50, 60, 70, 80, 90, 100, 200, 300, 400, 500, 600, 700, 800, 900, or 1000. In some embodiments, the equivalent concentration c_{eq}, of the second compound inside the reaction vessel is higher than the equivalent concentration c_{eq}, of the first compound inside the reaction vessel by a factor of 1.1, to 1000. 1.5 to 1000, 2 to 1000, 3 to 1000, 4 to 1000, or 5 to 1000.

In some embodiments, the excess of the second compound over the first compound inside the reaction vessel during step c) is essentially constant over time. The ratio of the equivalent concentration c_{eq}, of the second compound divided by the equivalent concentration c_{eq}, of the first compound may fluctuate by less than 15%, 14%, 13%, 12%, 11%, 10%, 9%, 8%, 7%, 6%, 5%, 4%, 3%, 2%, or 1%. In some embodiments, the concentrations of the first and the second compound inside the reaction vessel may be essentially constant over the duration of step c). The concentrations of the first and the second compound inside the reaction vessel may fluctuate by less than 15%, 14%, 13%, 12%, 11%, 10%, 9%, 8%, 7%, 6%, 5%, 4%, 3%, 2%, or 1%. A constant excess of the second compound over the first compound may be achieved by adding the second compound at the rate of its consumption plus the rate of loss in the permeate, if any. The rate of consumption of the second compound may, e.g., be calculated based on the rate of addition of the first compound into the reaction vessel, under the assumption that the reaction between the first and the second compound is fast relative to the rate of addition of both compounds. It is an advantage of the present invention over a continuous stirred reactor or a continuous reactor that is allows to maintain a high and constant concentration of the second compound, while avoiding an excessive use of reagents, in particular of the second reagent.

In some embodiments, the concentrations of the first and the second compound inside the reaction vessel are essentially constant over time. In some preferred embodiments, the equivalent concentration of the first compound may be close to zero, i.e. at or below the limit of detection, and the equivalent concentration of the second compound may be essentially constant during step c).

During step c), the content of the reaction vessel is subjected to membrane filtration so as to essentially retain the first compound, the second compound, and the third compound inside the reaction vessel. "Membrane filtration" is used herein to describe a pressure-driven separation process, which relies on the use of a semipermeable membrane allowing for "small" molecules, e.g. buffer and solvent molecules, to pass, but retaining "larger" molecules, e.g. the first, second and third compounds recited in the present method. The skilled person will understand that, while retention is strongly influenced by a compound's molecular weight, other factors such as conformation and chemical nature may also affect the compound's retention by the membrane. The liquid passing through the membrane is referred to as "permeate" or "filtrate", while the sample retained by the membrane is referred to as "retentate". As used herein, the expression "the content of the reaction vessel is subjected to membrane filtration" may describe a setup, where the retentate, i.e. the solution comprising the molecules unable to cross the membrane, is contained within or recycled to the reaction vessel. In other words: The reaction vessel is connected to a membrane filtration unit such that the content of the reaction vessel corresponds to the retentate of the membrane filtration process. As will be elaborated below with respect to the apparatus of the present invention, different setups may be used in order to subject the content of the reaction vessel to membrane filtration. For example, the content of the reaction vessel may be subjected to filtration through a membrane integrated into the reaction vessel (dead-end configuration). The flow through the membrane may be driven by applying nitrogen pressure to the solution inside the reaction vessel. Alternatively, the content of the reaction vessel may be subjected to membrane filtration by employing a membrane filtration unit and a liquid conduit allowing to cycle liquid/retentate from the reaction vessel via the membrane filtration unit back into the reaction vessel (cross-flow configuration). Together with any associated devices such as pumps and valves, the liquid conduit and the membrane filtration unit may be referred to collectively as a retentate loop. In one embodiment, the liquid conduit emerges from an outlet of the reaction vessel and leads via the membrane filtration unit into a dedicated inlet of the reaction vessel. Flow within the retentate loop may be driven by a pump, which, together with a pressure control valve, generates the pressure driving the formation of permeate. In such a setup, only the membrane filtration unit and a part of the retentate loop may be pressurized, while the contents of the reaction vessel are not subjected to increased pressure. Depending on the composition of the reaction mixture and the molecular weight cut-off of the membrane filtration unit, it is possible that the retentate is also enriched in further compounds in addition to the first, the second, and the third molecule.

For the purpose of the present invention, it is preferred to use membranes, which are essentially inert towards the compounds they get in touch with, e.g. compatible with acids, bases, and organic solvents. Depending on the application, an ultrafiltration membrane or a nanofiltration membrane may be used, e.g. membranes having a molecular weight cut-off of not more than 3 kDa, e.g. 3 kDa, 2 kDa, 1 kDa, 0.5 kDa, 0.2 kDa, 0.1 kDa, or below may be useful. For some examples, membranes having a molecular weight cut-off of not more than 10 kDa, e.g. 9 kDa, 8 kDa, 7 kDa, 6 kDa, 5 kDa, 4 kDa, may be useful (e.g. in a diafiltration step). For example, a ceramic or polymeric membrane with a molecular weight cut-off of less than 0.2 kDa may be used in many cases. It should however be understood that, as long as it provides a suitable molecular weight cut-off, the membrane comprised may be of any material known in the context of filtration, such as, e.g., polymer (e.g., nylon, polystyrene), metal, alloy, glass, ceramics, metal oxides, cellophane, cellulose, or composite material. The filter/membrane may be hydrophobic or hydrophilic. The surface of the membrane may be neutral or positively charged or negatively charged. As used herein, a filtration process is said to "essentially retain" a compound, if the rejection rate of the membrane employed is at least 90 % (= between 90% and 100%). The skilled person is aware of how to calculate the rejection rate [rejection rate=1-(concentration of compound inside the permeate/concentration of compound inside the retentate)]. A membrane may be used, which has a rejection rate of at least 91%, 92%, 93%, 94%, 95%, 96%, 97%, 98%, or 99% with respect to at least one of compounds 1, 2, and 3. Preferably, a membrane may be used, which has a rejection rate of at least 91%, 92%, 93%, 94%, 95%, 96%, 97%, 98%, or 99% with respect to each of compounds 1, 2, and 3. It is to be understood that, depending on the molecular weight, the rejection of compounds 1, 2, 3 may differ. For example, the rejection rate may be 99% for compounds 1 and 3, and 90% for compound 2. To avoid clogging of membrane pores, it may be preferable to employ a tangential flow filtration format (aka. cross flow filtration). For example, cross-flow nanofiltration may be used. As explained above, in this setup, the content of the reaction vessel is conducted to a cross-flow membrane filtration unit and the retentate from said cross-flow membrane filtration unit is conducted back into the reaction vessel. The skilled person will readily understand that the compounds "retained in the reaction vessel" are contained in the liquid/retentate, which is cycled through the reaction vessel and the retentate loop. In a dead-end filtration setup, when the filtration membrane is integrated in a side wall of the reaction vessel, the retentate may be said to be withheld within the reaction vessel.

The membrane filtration process applied during step c) allows keeping the volume of liquid inside the reaction vessel low over the duration of step c), even if a comparatively large volume of the solution comprising the first compound is added. This may be achieved by the constant withdrawal of permeate from the contents of the reaction vessel. Preferably, the volume of liquid inside the reaction vessel is kept essentially constant over the duration of step c). For instance, the volume may deviate from and fluctuate around a given set value by less than 30%, less than 20%, less than 15%, less than 10%, less than 5%, or even less than 2% of said value. Preferably, membrane filtration is carried out during the whole duration of step c). However, membrane filtration may be interrupted during step c), started after the beginning of step c), or ended before the end of step c). In some embodiments, membrane filtration is carried out over at least 70%, at least 80%, or at least 90% of the duration of step c).

In some embodiments, the method according to the present invention may additionally comprise the addition of a liquid, which is miscible with the reaction mixture, into the reaction vessel while subjecting the content of the reaction vessel to membrane filtration. The solvent or solution added may correspond to the liquid reaction medium of step b), or may be an aqueous or organic solution. It may comprise organic solvents, buffers, and further reagents. Such a step may be carried out during step c), e.g. concomitantly or alternating with the addition of the solution S1 and/or with the addition of the solution S2. In connection with the filtration process, this may enable excellent control over the composition of the reaction mixture, e.g. over the concentrations of the first compound, the second compound, the third compound and any unwanted side products or impurities contained in the reaction mixture. For example, the filtration rate, the influx of solvent, the influx of the solution S1 (comprising the first compound), and the influx of the solution S2 (comprising the second compound) may be independently adjusted so as to control the composition of the reaction mixture and keep it within certain set values. For instance, in order to counteract a buildup of an unwanted compound, diafiltration may be carried out during step c). This may be achieved by increasing the filtration rate and compensating this by an increased influx of liquid reaction medium into the reaction mixture.

Depending on the specific circumstances, the membrane filtration process may be extended beyond step c). For example, membrane filtration may be carried out during the whole duration of step c) and be continued thereafter. If continuing membrane filtration after step c), it may be used to concentrate the product solution and/or for diafiltration (i.e. salt and or solvent exchange by addition of an alternative buffer or solvent during filtration). Further, membrane filtration may be employed after step c) in order to remove compounds, which would interfere with the further processing of the reaction product, e.g. residual amounts of either the first or the second compound. In such a setup, it may be desirable to use a membrane filtration unit with a different, e.g. higher, molecular weight cut-off for the part of the membrane filtration process, which is carried out after completion of step c). This may be achieved by using specific embodiments of the apparatus according to the present invention, which allow switching between different membrane filtration units during operation of the apparatus.

In some embodiments of the present invention, the third compound formed by reaction of the first and the second compound is metastable. As used herein, "metastable" may mean that the lifetime of the third compound is limited. For example, the third compound may undergo a spontaneous intramolecular reaction, thereby forming a cyclic fourth compound.

In some embodiments, the third compound is incubated with a fifth compound, thereby forming a sixth compound. This may occur subsequently to step c) or subsequently to further diafiltration or concentration steps. Alternatively, the reaction with the fifth compound may occur during step c), and the fifth compound may be provided dissolved in the liquid reaction medium of step b) and/or added to the reaction mixture as part of one of the solutions S1 and S2.

In some embodiments, the present invention thus provides a method for forming an intramolecular bond between two types of reactive groups of a precursor compound, thereby generating a cyclic target compound, the method comprising the following steps:
a') Providing a first solution S1 comprising the precursor compound and a second solution S2 comprising an activating reagent;
b') Providing, inside a reaction vessel, a solution S3 comprising the activating reagent in a liquid reaction medium, which comprises an organic solvent; and
c') Concomitantly adding the solutions S1 and S2 over an extended period of time into the solution S3 while mixing the content of the reaction vessel, thereby forming a reaction mixture comprising the cyclic target compound;
wherein:
d') The activating reagent is in excess over the precursor compound inside the reaction vessel; and
e') During step c'), the content of the reaction vessel is subjected to membrane filtration so as to essentially retain the precursor compound, the activating reagent, and the cyclic target compound inside the reaction vessel.

For the avoidance of doubt, the definitions, explanations, preferred embodiments, and further method steps laid out before are applicable to elements a') to e') of the above method of generating a cyclic target compound. In this embodiment of the method of the present invention, the activating reagent (i.e. the second compound) will react with a first type of reactive group of the precursor compound (i.e. the first compound), thereby forming a metastable third compound. This metastable third compound will then undergo an intra- or intermolecular reaction, which results in the formation of a chemical bond between the first type of active group and the second type of active group of the precursor compound. As an example, said chemical bond may selected from an amide bond, an ester bond, an ether bond, a thioester bond, or a phosphoester bond. Preferably, the formation of an intramolecular bond is favored, e.g. by slow dosing of the precursor compound into the reaction vessel, thereby keeping the concentrations of the precursor compound and the metastable third compound inside the reaction vessel close to zero.

In some embodiments of the above method of generating a cyclic target molecule, the precursor molecule is selected from a peptide, a protein, a peptide conjugate, a protein conjugate, a nucleic acid, or an analog of a nucleic acid.

In preferred embodiments, the cyclic target compound is a cyclic peptide. The method of the present invention allows carrying out the cyclization reaction in a highly controlled, reproducible way, thereby reducing oligomer formation.

Cyclic peptides may be differentiated according to their chemical structure, e.g. in terms of the bonds that are comprised in the ring. Generally speaking, cyclization may occur by chemical bond formation between functional groups located either on both ends of the molecule, on two side chains of the molecule, or on one end and one side chain of the molecule. In homodetic cyclic peptides, the ring consists of moieties linked by peptide bonds between the alpha amino group and the alpha carboxyl group. In cyclic isopeptides, the ring comprises at least one non-alpha amide linkage, e.g. involving a side chain amino group and/or a side chain carboxyl group. In cyclic depsipeptides, the ring comprises at least one ester linkage instead of the amide linkage, formed e.g. by reaction of the C-terminal carboxyl group with a side chain hydroxyl group. Other constellations, e.g. where the hydroxyl group is at one terminus and the carboxyl group at the other terminus or at a side chain or where both reactive groups are located at a side chain, are likewise possible. Depending on the structure of the desired cyclic target peptide, the skilled person will provide a suitably protected precursor peptide, where only the reactive groups to be involved in the cyclisation reaction are free, but other reactive groups are blocked from reaction by suitable protective groups. The first and the second reactive group may be located on either end of the precursor peptide, on two side chains of the precursor peptide, or on one end and one side chain of the precursor peptide. The skilled artisan is aware of numerous well-known synthetic strategies for producing partially protected peptides and will routinely deploy them in order to provide the solution of step a'). As has been noted above, the solution of step a') may be a solution of a crude, partially protected peptide.

In some embodiments, the first compound is a crude peptide and the second component is a carboxyl group activating reagent. In some embodiments, the first compound is a partially protected peptide and the second component is a carboxyl group activating reagent. In some embodiments, the first compound is a partially protected peptide and the second compound is [Ethylcyano(hydroxyimino)acetato-O²]tri-1-pyrrolidinylphosphonium hexa fluorophosphate (PyOxim). In some embodiments, the reaction medium comprises N,N-dimethylformamide (DMF).

In some embodiments, the present invention provides a method for coupling a peptide to an activated carrier compound, thereby generating a peptide carrier conjugate, the method comprising the following steps:
a") Providing a first solution S1 comprising the activated carrier compound and a second solution S2 comprising the peptide;
b") Providing, inside a reaction vessel, a solution S3 comprising the peptide in a liquid reaction medium, which comprises an organic solvent; and
c") Concomitantly adding the solutions S1 and S2 over an extended period of time into the solution S3 while mixing the content of the reaction vessel, thereby forming a reaction mixture comprising the peptide carrier conjugate;
wherein:
d") The peptide is in excess over the activated carrier compound inside the reaction vessel;
e") During step c"), the content of the reaction vessel is subjected to membrane filtration so as to essentially retain the activated carrier compound, the peptide, and the peptide carrier conjugate inside the reaction vessel.

For the avoidance of doubt, the definitions, explanations, preferred embodiments, and further method steps laid out before are applicable as well to elements a") to f") of the above method of generating a peptide carrier conjugate.

The activated carrier compound may be an activated polyethylene glycol (PEG) or PEG derivative, e.g. a linear or branched PEG Maleimide such as NHS-PEG-NHS. In such cases, if the coupling reaction between the peptide and the carrier molecule is not complete, the coupling reaction may yield a peptide carrier conjugate, which is a mixture of unconjugated carrier molecules, carrier molecules conjugated to the maximal amount of peptide molecules, and carrier molecules conjugated to less than the maximal amount of peptide molecules. Because of the large size of the PEG compared to the conjugated peptide, it may be very challenging to separate the desired species with the maximal amount of peptide from this mixture. It may therefore be essential for product purity to achieve a good coupling efficiency. This in turn may necessitate to perform the coupling reaction using a large excess of peptide. The method of the present invention is advantageous in this setting, because it allows to perform the coupling reaction under highly controlled and reproducible conditions. In particular, a high excess of peptide may be maintained during the coupling reaction in step c") of the method according to the present invention, but without losing a large quantity of unbound peptide. The method may analogously be applied to the coupling of active pharmaceutical ingredients other than peptides to an activated carrier compound.

It will be understood by a person skilled in the art that the optimal concentration of a peptide in the solutions of step a') or b") depends on the molecular properties of the peptide and the skilled person will routinely optimize this concentration. In general, the peptide concentration may be chosen so as to keep the volume of the solution of step a') or b") low while avoiding peptide aggregation and/or precipitation. In some embodiments, the concentration of the peptide in the solution of step a') or b") is chosen to be equal to or higher than 10, 20, 30, 40, 50, 60, 70, 80, 90, 100, 110, 120, 130, 140, 150, 160, 170, 180, 190, or 200 mmol/l. In other embodiments, the concentration of the peptide in the solution of a') or step b") is chosen to be equal to or higher than 10, 20, 30, 40, 50, 60, 70, 80, 90, 100, 110, 120, 130, 140, 150, 160, 170, 180, 190, or 200 g/l.

Preferably, the cyclic peptide or the peptide carrier conjugate produced according to the method of the present invention may be subjected to purification after completion of step c') or c") and any further diafiltration and/or concentration step performed. Means for purification and isolation, which may optionally be used in this context, comprise, e.g., crystallization, lyophilization, one or more electrophoretic methods (e.g., gel electrophoresis or capillary (CE) electrophoresis), one or more additional precipitation-based methods (e.g., salting in or salting out), one or more dialytical methods (dialysis), and/or one or more chromatographic methods (e.g., gel permeation chromatography (GPC), size exclusion chromatography, Ion exchange chromatography (IEC), high performance liquid chromatography (HPLC), reversed phase HPLC (RP-HPLC), fast protein liquid chromatography (FPLC), Flash Chromatography (flash), Rapid Refluid Liquid Chromatography (RRLC), Rapid Separation Liquid Chromatography (RSLC), Ultra Fast Liquid Chromatography (UFLC), reversed phase UFLC (RP-UFLC), Ultra Performance Liquid Chromatography (UPLC) or reversed phase UPLC (RP-UPLC) Preferably, the oxidized peptide is subjected to at least one dimension of reversed phase HPLC. The skilled person is well aware of the fact that the cyclic peptide or peptide carrier conjugate may need to undergo a step of protecting group removal before such purification steps. Where warranted, the skilled person will implement such a step of protection group removal as part of her routine work.

In some embodiments, the present invention provides a method for activating a carrier compound, the method comprising the following steps:
a‴) Providing a first solution S1 comprising the carrier compound and a second solution S2 comprising an activating reagent;
b‴) Providing, inside a reaction vessel, a solution S3 comprising an activating agent in a liquid reaction medium, which comprises an organic solvent; and
c‴) Concomitantly adding the solutions S1 and S2 over an extended period of time into the solution S3 while mixing the content of the reaction vessel, thereby forming a reaction mixture comprising activated carrier compound;
wherein:
d‴) The activating agent is in excess over the carrier compound inside the reaction vessel;
e‴) During step c‴), the content of the reaction vessel is subjected to membrane filtration so as to essentially retain the carrier compound, the activating reagent, and the activated carrier compound inside the reaction vessel.

In some embodiments, the method further comprises a step of:
f‴) Subjecting the content of the reaction vessel to diafiltration after conclusion of step c‴), wherein the molecular weight cut-off is chosen such that the activated carrier compound is retained inside the reaction vessel, but the activating reagent is removed.

A further aspect A1 of the present invention relates to an apparatus suitable for performing the method according to the present invention, comprising:
A. a reaction vessel, which comprises at least two inlets and is equipped with a means for mixing the contents of the vessel;
B. two feed tanks, each connected to one of the inlets of the reaction vessel; and
C. means allowing to effect and control liquid flow from the feed tanks via the inlets into the reaction vessel;
D. two membrane filtration units, each having a retentate side and a permeate side, which are connected to the reaction vessel in such a way that the liquid on the retentate side of each of the membrane filtration units remains inside the reaction vessel or is cycled back into the reaction vessel.

In a further aspect A2, the apparatus according to aspect A1, further comprises a third feed tank (10) connected to the reaction vessel via a liquid line.

In a further aspect A3, the apparatus according to any one of aspects A1 to A2, further comprises a means for determining the volume of liquid contained in or recycled through the reaction vessel.

A further aspect A4 relates to the apparatus according to any one of aspects A1 to A3, wherein both membrane filtration units are in cross flow configuration.

A further aspect A5 relates to the apparatus according to any of aspects A1 to A4, wherein said means for determining the volume of the liquid contained in or recycled through the reaction vessel generates a feedback signal used to control the filtration rate through the membrane and/or the addition rate of liquid from at least one of the storage vessels (2, 3, 7) into the reaction vessel.

A further aspect A6 relates to the apparatus according to any one of aspects A1 to A5, wherein the two membrane filtration units differ with respect to the molecular weight cut-off of their membranes.

Preferably, all materials the apparatus is made of are (essentially) inert against the reagents they will be exposed to. Preferably, the material is compliant with the applicable regulations for the production of pharmaceutical products, cosmetics and/or food and beverages, i.e. it preferably complies with good manufacturing practices (GMP). Further, to minimize the risk of electrostatic ignition, an electrically conductive material may be used.

Herein, the expression "reaction vessel" or "reactor" is used for a container, which is suitable to take up the reagents of interest, and which preferably has at least one liquid outlet. The reaction vessel may be shaped so as to enable efficient mixing of its contents and rinsing of its walls. The reaction vessel will preferably be closed, i.e. suitable to avoid contamination or unintended release of its contents.

The reaction vessel may be made of any suitable material e.g. of metals, glass, enamel, or polymers such as polypropylene, polyethylene, polyvinyl chloride, polystyrene, and polyether ether ketone. Preferably, a material is chosen, which is essentially inert against the reagents it will be exposed to. Further, to minimize the risk of electrostatic ignition, an electrically conductive material may be used. In some embodiments, the reaction vessel is made of stainless steel or Hastelloy alloys or of glass. The size and dimensions of the reaction vessel can be chosen according to the scale intended. For example, reactors with an inner volume of (about) 5 to (about) 100 liters, of (about) 20 to (about) 250 liters, of (about) 30 to (about) 200 liters, of (about) 40 to (about) 150 liters, of (about) 50 to (about) 100 liters, or of (about) 60 to (about) 75 liters may be used. For example, reactors with an inner volume of more than 10, 20, 30, 40, 50, 75, 100, 150, 200, or 250 liters may be used. In some embodiments, the reaction vessel has an inner volume of (about) 10 to (about) 30 liters.

The reaction vessel may be adapted for working under protective atmosphere. For example, the reaction vessel(s) may comprise a first controllable valve, which may be connected to a vacuum source, a second controllable valve, which may be connected to a source of inert gas such as nitrogen, and an electronic or mechanical pressure controller. Preferably, the valves may be operated automatically, e.g. by a central control unit. In other embodiments, the reaction vessel comprises merely a controllable valve, which can be connected to a source of inert gas such as nitrogen, and allows overlaying the vessel's liquid content with protective gas.

In order to enable temperature control, the reaction vessel may be a jacketed reactor. Preferably, a temperature sensor inside the reactor provides a feedback signal controlling the circulation of cooling or heating fluid inside the reactor's jacket.

As has been explained above with respect to the methods of the present invention, any suitable mixing device may be used to mix the contents of the reaction vessel. In some embodiments, a stirrer inside the reaction vessel may be used. In other embodiments, a pumping circuit may be used to mix the contents of the reaction vessel. This pumping circuit may overlap with or be identical with the retentate loop delivering liquid from the reaction vessel to the membrane filtration unit and recycling retentate from the membrane filtration unit back into the reaction vessel. The retentate loop may comprise a liquid outlet allowing the withdrawal of a steady stream of reaction product. Such a setup may be used in a continuous process as opposed to a batch process.

The reaction vessel comprises at least two inlets for liquid flow, which are each connected to at least one feed tank. Said inlets may be spatially separated and may be positioned such that the respective liquid may be added from the top of the reactor onto the liquid comprised therein, or such that the liquid may be added from below the liquid surface. The reaction vessel may comprise additional inlets and outlets for materials, preferably for liquids. For example, the reaction vessel comprises at least one further liquid inlet, which allows introducing the recycled retentate into the reaction vessel. In another example, the reaction vessel may comprise at least one further inlet, which allows introducing a further liquid, e.g. a liquid reaction medium or a buffer for diafiltration, into the reaction vessel. This latter inlet may be connected to a spray head inside the reactor. Any inlets and outlets may comprise a valve, which is closed unless material is purposefully being introduced into or let out of the reaction vessel. Preferably, material transfer through the inlets and outlets is driven and controlled by automatic devices (e.g. pumps and valves), which may be governed by a central or local control unit.

In some embodiments, the present apparatus may comprise a third feed tank connected to the reaction vessel via a liquid line. The connection to the reaction vessel may be realized by different configurations. For example, the reaction vessel may comprise three inlets, each connected to a separate feed tank. Alternatively, the first and/or the second inlet of the reaction vessel may be connected to more than one feed tank. Such a setup may be useful, e.g., in order to dilute one of the reagent influxes with liquid reaction medium during dosing into the reaction vessel.

Herein, the expression "feed tank" may be used for a container, inside of which a material of interest can be stored under suitable conditions. The skilled person will be able to define suitable conditions depending on the circumstances, e.g. so as to preserve the material's integrity or process safety according to the specifications of a given process. The feed tank(s) may be made of any suitable material, e.g. of metals, glass, enamel, or polymers such as polypropylene, polyethylene, polyvinyl chloride, polystyrene, and polyether ether ketone. In some embodiments, the feed tank is made of stainless steel or Hastelloy alloys. The size of the feed tank may be chosen according to the scale of synthesis intended. In some embodiments, the feed tank has an inner volume of more than 0.5 to 50 liters, e.g., at least 0.5, 1, 2, 3, 4, 5, 10, 15, 20, 25, 30, 35, 40, 45, or 50 liters. In some embodiments, the feed tank has an inner volume of (about) 0.5 to (about) 20 liters.

In order to enable temperature control, the feed tank(s) may be jacketed. Preferably, a temperature sensor inside the reactor provides a feedback signal controlling the circulation of cooling or heating fluid inside the vessel's jacket.

In some embodiments, the feed(s) tank may further be adapted to contain a protective gas. For example, the vessel may comprise a pressure controllable valve, which can be connected to a vacuum source, and with a second controllable valve, which can be connected to a source inert gas such as nitrogen. In some embodiments, the flow of liquid from the feed tank(s) may be driven by the pressure resulting from applying nitrogen to the feed tank.

The feed tanks are each connected to an inlet of the reaction vessel. The connection may be by means of a conduit. Any available means allowing to effect and control liquid flow from the feed tanks via the inlets into the reaction vessel may be used. Typically, liquid flow out of the feed tanks may be driven by gravity, by pressurizing the feed tank, by applying vacuum suction to the reaction vessel, or by means of a pump. Moreover, flow into the reaction vessel may be controlled by a valve. For example, liquid flow from the feed tanks into the reaction vessel may be effected and controlled by pumps, which may be referred herein as dosing pumps. The skilled person will routinely determine the specifications of the dosing pumps in dependence of the dimension of the specific apparatus at hand and the anticipated volumes of liquid to be dosed into the reaction vessel.

The membrane filtration units of the present apparatus may comprise a membrane housing, which may have at least one outlet for the permeate. The cross-flow membrane filtration units of the present apparatus may comprise a membrane housing, which may have one feed inlet and one outlet each for the permeate and the retentate. As used herein, "retentate" refers to the part of the solution, which is retained, i.e. does not cross the membrane during the filtration process. Hence, as used herein, the "retentate side" refers to that side of the membrane, where the retentate is to be found. Analogously, as used herein, "permeate" refers to the part of the solution, which passes through the membrane, i.e. does cross the membrane during the filtration process. Hence, as used herein, the "permeate side" refers to that side of the membrane, where the permeate emerges. The membrane housing may be sealed and hold a membrane module. The skilled artisan may routinely choose a suitable membrane module depending on the specifications of the chemical reaction to be carried out (e.g. the products to retain and to filter out) and the apparatus at hand. Common examples include tubular modules made of multi- or monochannel ceramic membranes, spiral wound modules made of polymeric membranes, membrane cassettes or hollow fiber. Metal or composite membranes may likewise be used. The membrane surface area may be dimensioned in order to achieve a desired operation time.

The membrane filtration units are connected to the reaction vessel in such a way that the liquid on the retentate side of each of the membrane filtration units remains inside the reaction vessel or is cycled back into the reaction vessel. The former may occur when using a membrane filtration unit, which is integrated into the bottom wall of the reaction vessel (dead end configuration). The latter may occur when using a membrane filtration unit in cross-flow configuration. "Cross-flow configuration" may refer to a setup, where the majority of the feed flow travels tangentially across the surface of the membrane. The permeate formation may then occur across the membrane, i.e. in a direction perpendicular to the direction of the feed flow.

Various configurations of the apparatus according to the present invention may be envisaged. For example, one of the membrane filtration units may be integrated into the bottom wall of the reaction vessel (dead end configuration) and one membrane filtration unit may be connected to the reaction vessel via a circuit of liquid lines (cross flow configuration). In such a case, the reaction vessel may be pressurized in order to drive the liquid flow through the membrane filtration unit, which is integrated into the reaction vessel's wall. This then requires the use of suitable dosing pumps to effect liquid flow into the reaction vessel against pressure.

Alternatively, both membrane filtration units may be in cross flow configuration. In this case, an outlet of the reaction vessel may be connected via a liquid line to an inlet of a first membrane filtration unit, and the retentate outlet of the first membrane filtration unit may be connected to an inlet of the reaction vessel. Such a retentate loop may further comprise a circulation pump driving liquid circulation and formation of the permeate, a pressure control valve, and pressure sensors before and after the membrane filtration unit. The second membrane filtration unit may be connected to the reaction vessel by a second, completely independent retentate loop. Alternatively, the two membrane filtration units may be arranged in parallel inside the same retentate loop, such that they share the same circulation pump, pressure control valve, and pressure sensors, and a part of the afferent and efferent liquid lines. The liquid line(s) connecting the retentate outlet of the membrane filtration unit(s) to the reaction vessel may lead to a dedicated inlet of the reaction vessel. Alternatively, said liquid lines may lead to the same inlet as is used for the liquid from one of the feed tanks. In other words, the retentate loop may emerge from an outlet of the reaction vessel and lead via the membrane filtration unit into the same reaction vessel inlet as one of the feed tanks. In this case, said feed tank may be connected to said inlet via the retentate loop, i.e. a liquid conduit emerging from the feed tank discharges into the retentate loop. In such a configuration, the flux of the retentate loop is used to pre-dilute the liquid from the feed tank before it enters the reaction vessel.

The pressure driving permeate formation may be obtained by applying nitrogen pressure to the reaction vessel. Alternatively, the flux of liquid through the retentate loop may be effected by a recirculation pump integrated into the retentate loop. The pump may preferably be dimensioned such as to ensure a linear velocity of 1 to 5 m/s on the membrane surface. The pressure inside this loop may be regulated via the pump performance and by means of a pressure control valve integrated into the retentate loop. The formation of permeate may be driven by the pressure applied to the liquid. The permeate may be discharged from the membrane filtration unit. In some embodiments, a retentate loop may further comprise a valve positioned immediately before the inlet into the reaction vessel, a second pump and a liquid connection line. Said liquid connection line branches from the afferent liquid line, which goes from the retentate outlet of the membrane filtration unit to the reaction vessel, and leads to the efferent liquid line, which goes from the outlet of the reaction vessel to the inlet of the membrane filtration unit. One pump is integrated into this efferent liquid line between the reaction vessel's outlet and the intersection with the connecting line, and one pump is integrated into this efferent liquid line between the intersection with the connecting line and the inlet to the membrane filtration unit. The skilled person is well aware that such an arrangement may be used to uncouple the flux of liquid from the reaction vessel, which may be driven by the first pump, from the flux occurring inside the membrane filtration unit, which may be driven by a (stronger) recirculation pump. In such an arrangement, it may be possible to alter, in particular increase, the filtration rate, without having to alter the rate, at which liquid flows from the reaction vessel and re-enters the reaction vessel.

For the purpose of the present invention, it is preferred to use membranes for nano- and ultrafiltration as has been detailed above with respect to the methods of the present invention. Membranes having a molecular weight cut-off of not more than 10 kDa, e.g. 9 kDa, 5 kDa, 3 kDa, 2 kDa, 1 kDa, 0.5 kDa, 0.2 kDa or below may be used. In a preferred embodiment, a ceramic or polymeric membrane with a molecular weight cut-off of less than 0.5 kDa is used. It should however be understood that, as long as it provides a suitable molecular weight cut-off, the membrane may be of any material known in the context of filtration, such as, e.g., polymer (e.g., nylon, polystyrene), metal, alloy, glass, ceramics, metal oxides, cellophane, cellulose, or composite material. The membrane may be hydrophobic or hydrophilic. The surface of the membrane may be neutral or positively charged or negatively charged. It is preferred to use membranes compatible with acids, bases, and organic solvents, and which are stable at the pressure and temperature used. Likewise, the housing and sealing of the membrane filtration unit are preferably stable against these influences. The two membrane filtration units of the apparatus according to the present invention may be the same or different. For example, if two membrane filtration units with the same properties are used, the apparatus of the present invention may allow to regenerate one membrane, while using the other for filtration and vice versa. This may facilitate to process material prone to cause membrane clogging without frequent interruptions to the filtration process. Alternatively, the two membrane filtration units may be chosen to have different filtration characteristics. Hence, the two membrane filtration units may differ with respect to the molecular weight cut-off of their membranes. For example, one membrane may be a nanofiltration membrane, which is used during the reaction of the first compound with the second compound to withhold the educts and products of interest; the second membrane may be a microfiltration unit used in a subsequent diafiltration step in order to perform a solvent exchange and/or to remove compounds with a relatively small molecular weight from the product obtained.

The present apparatus may further comprise dedicated rinsing lines for applying a cleaning liquid to the membrane filtration units and for draining the cleaning liquid from the membrane filtration units. These rinsing lines may be connected by means of a multiway valve to the liquid line leading into each membrane filtration unit and to the liquid lines emerging from the outlets of each of the membrane filtration units.

To allow for temperature control within the retentate loop, the retentate loop may be equipped with a heat exchanger, such as with a shell and tube heat exchanger, a plate heat exchanger, a plate and frame heat exchanger, or a spiral plate heat exchanger. The flow of the heating/cooling medium within the heat exchanger may be in the same direction as the flow of the retentate inside the retentate loop (concurrent flow), against it (counter flow), or intersecting with it (crossflow). Hybrid configurations such as cross counterflow and multi pass flow are likewise possible. The skilled person will routinely choose a heat exchanger of suitable type and dimensions to achieve the heating/cooling needed.

The apparatus according to the present invention may further comprise a means for determining the volume of liquid contained in or recycled through the reaction vessel. As used herein, "the volume of liquid contained in or recycled through the reaction vessel" refers to the liquid volume contained inside the reaction vessel plus the liquid volume inside any retentate loop, which is connected to the reaction vessel and is in operation at the time of measurement. The skilled person is well aware of various, alternative options for determining said volume. It may, e.g., be determined by means of a level sensor placed inside or outside the reaction vessel. As further examples, the volume may be determined by a scale for weighing the reaction vessel, or by determining the volume or weight of the permeate removed from the reaction vessel. As density of the permeate and the volume of liquids added into the reaction vessel and retentate loop are known, the actual volume may then be calculated by subtracting the volume of the permeate.

In one embodiment, said means for determining the volume of the liquid contained in or recycled through the reaction vessel generates a feedback signal used to control the filtration rate through the membrane and/or the addition rate of liquid from at least one of the feed tanks into the reaction vessel. Such feedback loops may allow to keep the volume inside the reaction vessel essentially constant. In some embodiments, where a process solvent or liquid reaction medium is added to the reaction vessel from a third feed tank, it becomes possible to keep the volume of liquid contained in or recycled though the reaction vessel constant, while also keeping the concentrations of the first and the second compound essentially constant over the duration of step c) of the present method. The concentrations of the first and second compounds inside the reaction vessel may be calculated based on their influxes into the reaction vessel, the consumption by the reaction, and the volume of the liquid inside or recycled through the reaction vessel. Alternatively, the concentrations of the first and second compounds inside the reaction vessel may be measured by a monitoring system, e.g. using IR or UV-Vis spectrometry.

The monitoring system may comprise, e.g., an optical probe with a suitable spectral range, such as dip probes for UV, Vis, NIR, IR (e.g. Raman probes), or an oxidation reduction potential probe, e.g. a suitable Pt or Au electrode. As an alternative to the use of dip probes, said sensors may be in the form of a flow cell and may be integrated into the retentate loop or in a bypass loop in those embodiments, where the reaction vessel is part of a recirculation circuit. In each of the aforementioned embodiments, the monitoring system may be used to follow any parameter suitable to determine the concentration of the reduced peptide, the concentration of the total peptide, the concentration of the oxidation reagent, the concentration of any other products or educts of the oxidation reaction, and/or a ratio between any of the foregoing. In particular, optical properties, conductivity, or the redox potential of the solution inside the reaction vessel may be determined by the monitoring system. One or more monitoring systems, e.g. an optical probe and a redox probe, may be used to follow several parameters in parallel.

A further aspect of the present invention relates to an apparatus suitable for performing the method according to the present invention, the apparatus comprising:
A. a reaction vessel, which comprises at least two inlets and is equipped with a means for mixing the contents of the vessel;
B. three feed tanks, each connected to one of the inlets of the reaction vessel;
C. pumps allowing to effect and control liquid flow from the feed tanks via the inlets into the reaction vessel;
D. two cross-flow membrane filtration units, each having a retentate side and a permeate side, which are connected to the reaction vessel in such a way that the liquid on the retentate side of each of the membrane filtration units is cycled back into the reaction vessel;
E. at least one pump driving circulation of the liquid from the reaction vessel to the membrane filtration unit and back into the reaction vessel;
F. a pressure control valve; and
G. a means for determining the volume of liquid contained in or recycled through the reaction vessel;

Another aspect of the present invention relates to an apparatus suitable for performing the method according to the present invention, the apparatus comprising:
A. a reaction vessel, which comprises at least two inlets and is equipped with a means for mixing the contents of the vessel;
B. three feed tanks, each connected to one of the inlets of the reaction vessel;
C. automated pumps allowing to effect and control liquid flow from the feed tanks via the inlets into the reaction vessel;
D. two cross-flow membrane filtration units, each having a retentate side and a permeate side, which are connected to the reaction vessel in such a way that a retentate loop is formed, where the liquid on the retentate side of each of the membrane filtration units is cycled back into the reaction vessel;
E. at least one automated recirculation pump and a pressure control valve integrated into the retentate loop;
F. a means for determining the volume of liquid contained in or recycled through the reaction vessel; and
G. a control unit, which receives input from at least the means for determining the volume of liquid contained in or recycled through the reaction vessel, and which uses this input to regulate the action of the pumps and of the pressure control valve.

Temperature control within the apparatus of the present invention may be achieved by the combined use of one or two jacketed feed tank(s), a jacketed reaction vessel, and a heat exchanger integrated into the retentate loop. In another embodiment, temperature control within the apparatus of the present invention may be achieved by the combined use of one or two jacketed feed tank(s) and a jacketed reaction vessel. In another embodiment, temperature control within the apparatus of the present invention may be achieved by the combined use of one or two jacketed feed tank(s) and a heat exchanger integrated into the retentate loop. In another embodiment, temperature control within the apparatus of the present invention may be achieved by the combined use of a jacketed reaction vessel and a heat exchanger integrated into the retentate loop. These temperature control means may be chosen such that the temperature of the liquids within the apparatus may be controlled to any desired temperature selected from the range of -10°C to 50°C, e.g. 5 to 50°C or 10°C to 30°C, preferably with a precision of +/- 1°C. In another embodiment, the temperature control means may be chosen such that the temperature of the liquid within the reaction vessel may be controlled to any desired temperature selected from the range of 5°C to 50°C.

The present apparatus may be used in a semi-continuous format as has been detailed above, where continuous feeds of the two compounds are pumped into the reaction vessel for a certain time.

The apparatus of the present invention provides for numerous options to control the reaction conditions inside the reaction vessel. As has been detailed above, it enables to control the concentrations of the educts and the desired product inside the reaction vessel (e.g. by modulating the feed rate of reagents into the reaction vessel and/or the membrane filtration rate, i.e. the rate of permeate formation), the temperature (e.g. by modulating the flux of the heating/cooling medium inside vessel jackets and heat exchangers) and pressure (e.g. by controlling the recirculation pump, the pressure control valve, or the stream of nitrogen applied), and the concentration of low molecular compounds (e.g. by modulating the membrane filtration rate, i.e. the rate of permeate formation). Preferably, the regulation of said parameters is performed by one or more automated devices, which receive input from appropriate sensors inside the reaction vessel or inside the recirculation loop. Preferably, said sensors comprise a level probe, pressure sensors, flowmeters, and temperature sensors. Further, one or more sensors selected from the group of a conductivity probe, a redox probe, and an optical probe (e.g. a UV-Vis probe or a NIR probe) may be included.

The automated controlling devices may be local or be part of a central control unit. Said central control unit may be organized to form different hierarchical levels of control, as may be the case for a supervisory control and data acquisition (SCADA) control system architecture. For example, the control unit may comprise one or more remote supervisory computers, which gather data from and send control commands to peripheral devices, and one or more peripheral devices such as remote terminal units (RTU), programmable logic controllers (PLC) and user interfaces such as GUI panels. The PLCs and the supervisory SCADA software may receive input from field sensors such as sensors of temperature, pressure, flow rate, and volume, amongst others. The one or more SCADA supervisory computing platform may additionally interact with a manufacturing execution system (MES), which in turn interacts with an enterprise resource planning (ERP) system. Moreover, the one or more SCADA supervisory computing platform may execute logging tasks by sending specific process parameters to a dedicated database. In some embodiments, the control unit comprises at least one SCADA system, at least one PLC with sensors and actors controlling the actions of the dosing pumps, the recirculation pump, and the pressure control valve. In some embodiments, the control unit further controls the draining of liquid from the reaction vessel, the influx of additional reagents, e.g. of diafiltration butter, into the reaction vessel, and the flow of heating/cooling fluid through vessel jackets and/or the retentate loop's heat exchanger. In some embodiments, the control unit may further control the flow of protective gas (e.g. of nitrogen) into the reaction vessel and the mixing device(s) of the reaction vessel.

In some embodiments, the control devices may be adjusted so as to keep the volume of liquid in the reaction vessel and the recirculation loop constant within a certain range. The skilled person will immediately recognize that, under this condition, the feed flows, the filtration rate and the rate of retentate removal (if applicable) will be interdependent. For instance, an increase of the concentration of the second compound inside the reaction vessel may be regulated by reducing the influx of the second solution S2 while compensating for this by either reducing the filtration rate and/or by increasing the influx of the second solution S1 and/or by dosing a process solvent/reaction liquid into the reaction vessel. The skilled person will determine which parameters are particularly influential for a specific reaction at hand and will configure the system so as to control them.

It should be noted that the previous explanations and descriptions of embodiments of the process according to the present invention are likewise applicable to the apparatus according to the present invention, and vice versa.

The following Figures and Examples, including the experiments conducted and the results achieved are provided for illustrative purposes only and are not to be construed as limiting to the scope of the claims.

### Detailed Description of the Figures

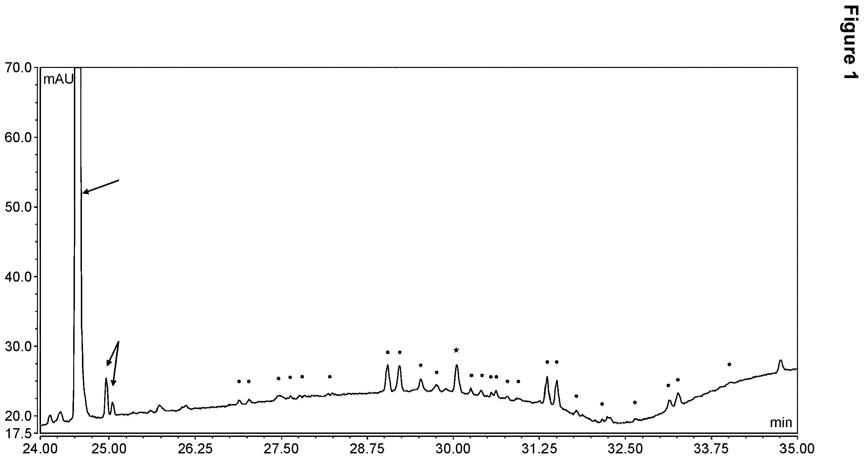

**Figure 1****:** Analytical chromatogram of the sample from example 1, experiment no 1. The peaks quantitated as monocyclic peptide (arrows), cyclic dimer (star) and other side products (dots) were considered for the analysis of the cyclisation reaction performance.

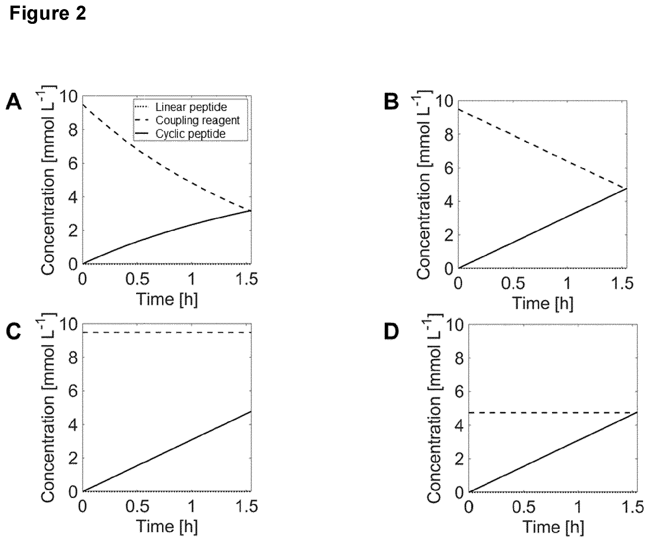

**Figure 2****:** Theoretical concentration profiles of reactants and products inside the reaction vessel during the cyclization of a model peptide, as detailed in example 9. Panel A: case study 9.1 (addition of peptide only into excess of coupling reagent inside the reaction vessel; no membrane filtration). Panel B: case study 9.2 (addition of peptide only into excess of coupling reagent inside the reaction vessel; concomitant membrane filtration). Panel C: case study 9.3 (addition of peptide and coupling reagent into excess of coupling reagent inside the reaction vessel; concomitant membrane filtration; high, constant excess of coupling reagent). Panel D: case study 9.4 (addition of peptide and coupling reagent into excess of coupling reagent inside the reaction vessel; concomitant membrane filtration; lower, constant excess of coupling reagent).

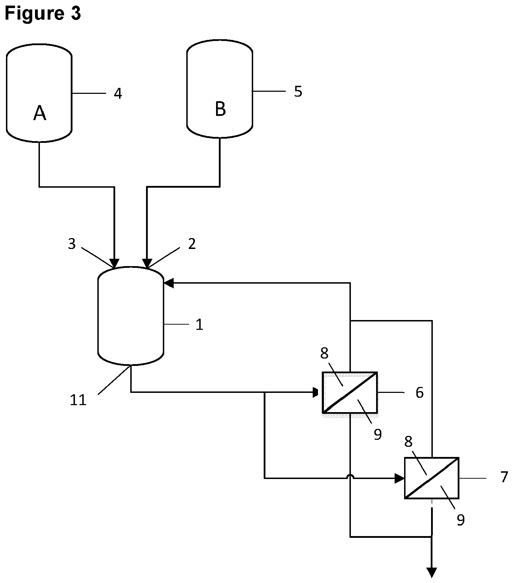

**Figure 3****:** Example of instrument setup with two membrane filtration units arranged in parallel inside the same retentate loop. A reaction vessel 1 comprising two liquid inlets 2,3 is connected via liquid lines to two upstream feed tanks 4,5. A liquid line connected to an outlet 11 of the reaction vessel 1 branches to serve two membrane filtration units 6, 7. The two liquid lines emerging from the retentate side 8 of each of the membrane filtration units 6,7 merge to one single line recycling retentate back to the reaction vessel 1. Likewise, the two liquid lines emerging from the permeate side 9 of the two membrane filtration units 6,7 merge to one single drain line. Multiway valves positioned at the "nodes" of the retentate loop (not shown) may be used to channel the liquid to either of the two membrane filtration units.

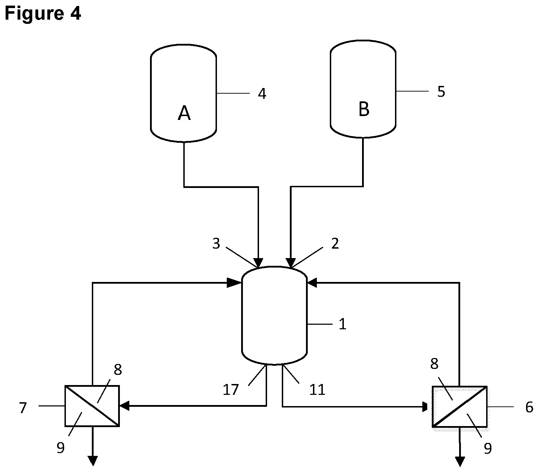

**Figure 4****:** Example of instrument setup with two membrane filtration units arranged in independent retentate loops. A reaction vessel 1 comprising two liquid inlets 2,3 is connected via liquid lines to two upstream feed tanks 4,5. Each of two membrane filtration units 6,7 is connected to the reaction vessel 1 via liquid lines directing liquid from a dedicated outlet 11, 17 of the reaction vessel to the membrane filtration unit and from the retentate side 8 of the membrane filtration unit back to the reaction vessel 1.

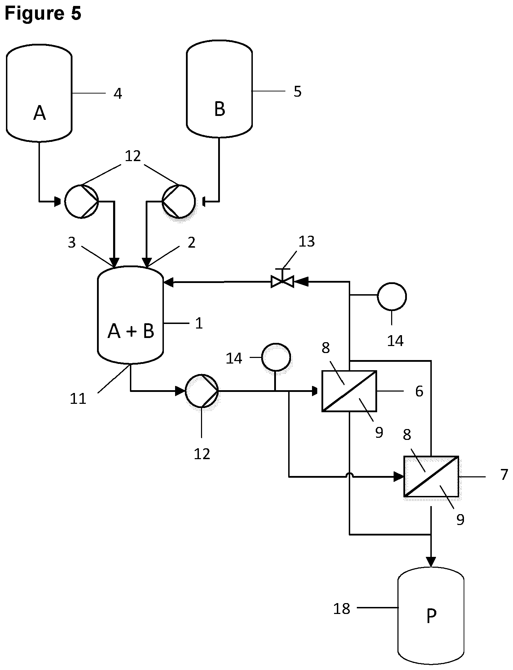

**Figure 5****:** Example of instrument setup with two membrane filtration units arranged in parallel inside the same retentate loop. A reaction vessel 1 comprising two liquid inlets 2,3 is connected via liquid lines to two upstream feed tanks 4,5. Pumps 12 drive and control liquid flow from the feed tanks 4,5 to the reaction vessel 1. A liquid line connected to an outlet 11 of the reaction vessel 1 branches to serve two membrane filtration units 6, 7. A pump 12 driving liquid circulation through the retentate loop is integrated into said line. The two liquid lines emerging from the retentate side 8 of each of the membrane filtration units 6,7 merge to one single line recycling retentate back to the reaction vessel 1. Likewise, the two liquid lines emerging from the permeate side 9 of the two membrane filtration units 6,7 merge to one single drain line leading to a container 18. A pressure control valve 13 and pressure sensors 14 are integrated into the retentate loop. Multiway valves positioned at the "nodes" of the retentate loop (not shown) may be used to channel the liquid to either of the two membrane filtration units.

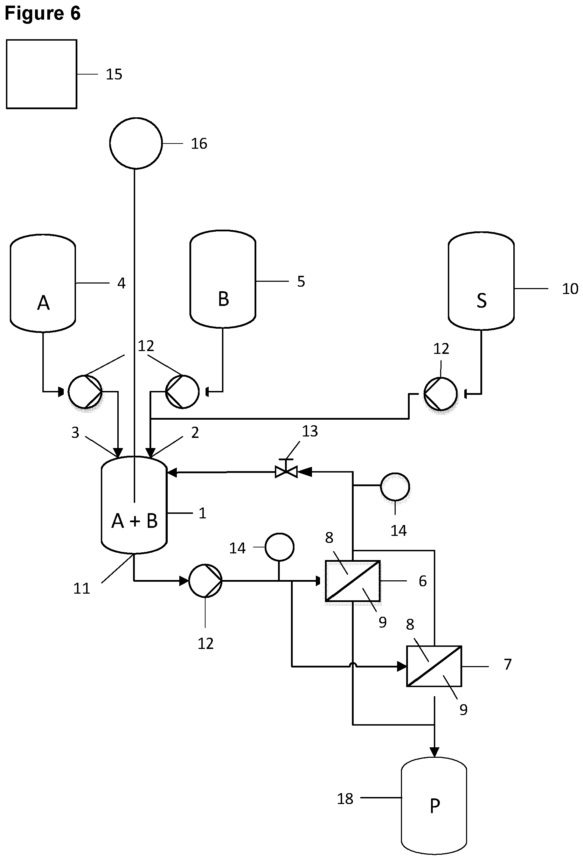

**Figure 6****:** Example of instrument setup with two membrane filtration units arranged in parallel inside the same retentate loop. A reaction vessel 1 comprising two liquid inlets 2,3 is connected via liquid lines to two upstream feed tanks 4,5. A third upstream feed tank 10 is connected to the liquid inlet 2 via the liquid line connecting feed tank 5 with liquid inlet 2. Pumps 12 drive and control liquid flow from the feed tanks 4,5,10 to the reaction vessel 1. A liquid line connected to an outlet 11 of the reaction vessel 1 branches to serve two membrane filtration units 6, 7. A pump 12 driving liquid circulation through the retentate loop is integrated into said line. The two liquid lines emerging from the retentate side 8 of each of the membrane filtration units 6,7 merge to one single line recycling retentate back to the reaction vessel 1. Likewise, the two liquid lines emerging from the permeate side 9 of the two membrane filtration units 6,7 merge to one single drain line leading to a container 18. A pressure control valve 13 and pressure sensors 14 are integrated into the retentate loop. Multiway valves positioned at the "nodes" of the retentate loop (not shown) may be used to channel the liquid to either of the two membrane filtration units. A level sensor 16 determines the volume of liquid inside the reaction vessel 1. A central control unit 15 receives input from at least the pumps 12, the pressure sensors 14, and the level probe. The input is used to control the actions of the pumps 12 and the pressure valve 13.

### List of Reference signs

- 1: reaction vessel
- 2: liquid inlet
- 3: liquid inlet
- 4: feed tank
- 5: feed tank
- 6: membrane filtration unit
- 7: membrane filtration unit
- 8: retentate side of membrane
- 9: permeate side of membrane
- 10: feed tank
- 11: liquid outlet
- 12: pump
- 13: pressure control valve
- 14: pressure sensor
- 15: central control unit
- 16: level sensor
- 17: liquid outlet
- 19: container

### Examples

### General procedure

Peptide 1 [H-Phg-D-Trp(Boc)-Lys(Boc)-Tyr(Bzl)-Phe-Hyp(2-Boc-aminoethyl-carbamoyl)-OH] was synthesized by SPPS using standard methods and Fmoc-amino acid derivatives. Peptide cleavage was performed using 30/70 HFIP/DCM. The raw peptide was precipitated from the cleavage cocktail using diisopropyl ether as anti-solvent. No further purification steps were applied.

The amounts of linear and cyclic peptide, as well as oligomers in the reaction mixture were determined by means of reversed-phase high-performance liquid chromatography (RP-HPLC). The samples were diluted to a concentration of approximately 1 g/l with respect to peptide and eluted using a gradient of 0.05% trifluoroacetic acid (TFA) in 1/99 v/v acetonitrile/water and 100% acetonitrile. The cyclisation performance was determined based on the relative amount of side products with respect to monocyclic peptide. LC-MS analysis was carried out to identify the peaks corresponding to peptidic products, such as monocyclic peptide and cyclic dimer. All the other peaks in the chromatogram of the reaction mixture, which did not appear in the pure solvent (baseline) or in the linear peptide samples, were assumed to correspond to other side products, including oligomers.

### Example 1: Cyclisation of peptide

Raw peptide 1 produced according to the general procedure was dissolved in DMF to a final peptide concentration of 125 g/l (91.6 mM). A round-bottomed flask equipped with a magnetic stirrer was charged with 20 ml of DMF solution containing either 119.4 mM or 59.7 mM PyOxim and a constant concentration of N,N-Diisopropylethylamine (DIPEA), proportional to the concentration of linear peptide used for the cyclisation. Subsequently, 10 ml of the peptide solution was gradually added at a constant flow rate of 2 ml/min using a liquid dosing pump. Samples were taken at the end of peptide addition and subjected to HPLC analysis. Experimental conditions and results are reported in Table 1.

**Table 1:**

| Exp. No. | c(PyOxim) [g/l (mM)] | V(DIPEA) [ml] | V(peptide sol. added) [ml] | Cyclic monomer [%] | Dimer [%] | Other side products [%] |
|---|---|---|---|---|---|---|
| 1 | 62.95 (119.4) | 0.625 | 10 | 93.38 | 0.85 | 5.74 |
| 2 | 31.48 (59.7) | 0.625 | 10 | 91.89 | 0.96 | 7.16 |

### Example 2: Cyclisation of peptide with decreasing excess of coupling reagent

The results from example 1 point to an advantageous effect of an excess of coupling reagent on the reaction performance. In the reaction scheme applied in example 1, the concentration of coupling reagent in the reaction mixture is expected to drop over time due to a) the dilution of the coupling reagent with the addition of new starting material in solution and b) its consumption during the coupling reaction. In order to assess the effect of this drop on the quality of the final product, experiments were carried out to compare the reaction mixture at the beginning of the reaction under the same reaction conditions as in example 1 (during the first 0.8 min of peptide addition: experiments 3 and 5 in Table 2) with the reaction mixture under the conditions at the end of the reaction as in example 1 (during the last 0.8 min of peptide addition: experiments 4 and 6 in Table 2). The experimental setup was essentially as in example 1. Experimental conditions and results are reported in Table 2.

**Table 2:**

| Exp. No. | c(PyOxim) [g/l (mM)] | V(DIPEA) [ml] | V(peptide sol. added) [ml] | Cyclic monomer [%] | Dimer [%] | Other side products [%] |
|---|---|---|---|---|---|---|
| 3 | 62.95 (119.4) | 0.1 | 1.6 | 98.9 | 0.53 | 0.57 |
| 4 | 36.50 (69.2) | 0.1 | 1.6 | 98.51 | 0.7 | 0.79 |
| 5 | 31.48 (59.7) | 0.1 | 1.6 | 98.19 | 0.7 | 1.09 |
| 6 | 5.04 (9.6) | 0.1 | 1.6 | 92.95 | 1.24 | 5.79 |

The results from Table 2 demonstrate that the formation of by-products increases with the decrease in concentration of coupling reagent (i.e. with the reaction time, compare experiment 3 with 4 and experiment 5 with 6). This effect appears particularly pronounced if starting with low concentrations of coupling reagent (compare experiment 4 and 6).

### Example 3: Cyclisation of peptide with constant concentration of coupling reagent

To evaluate whether the formation of by-products could be suppressed by keeping the concentration of coupling reagent constant over time, experiments 7 and 8 (see Table 3) were carried out to be compared with experiments 1 and 2, respectively (see Table 1). In experiments 7 and 8, 20 ml of a highly concentrated solution of the coupling reagent PyOxim in DMF was added simultaneously to peptide addition, using a second KNF SIMDOS^{®} 10 liquid dosing pump at a constant flow rate of 4 ml/min. The remaining experimental conditions were as in experiments 1 and 2, respectively. The concentration of the coupling reagent solution was selected so as to compensate for the drop in the concentration of coupling reagent by consumption and dilution. Experimental conditions and results are reported in Table 3.

**Table 3:**

| Exp. No. | c(PyOxim) [g/l (mM)] | V(DIPEA) [ml] | V(peptide sol. added) [ml] | c(PyOxim) in solution added [g/l (mM)] | Cyclic monomer [%] | Dimer [%] | Other side products [%] |
|---|---|---|---|---|---|---|---|
| 7 | 62.95 (119.4) | 0.625 | 10 | 125.90 (238.7) | 95.99 | 0.68 | 3.30 |
| 8 | 31.48 (59.7) | 0.625 | 10 | 78.69 (149.2) | 94.98 | 0.55 | 4.49 |

The results from Table 3 demonstrate that keeping the concentration of coupling reagent constant at the expense of having to increase the reaction volume helps to control the amount of dimers formed, but is not satisfactory with respect to the formation of other side products.

### Example 4: Cyclisation of peptide with increasing reaction volume

In order to assess the effect of increasing the reaction volume on the reaction performance, experiments 9 and 10 (see Table 4) were carried out to be compared with experiments 7 and 8, respectively (see Table 3). In experiments 9 and 10, instead of 20 ml of PyOxim in DMF, 20 ml of the solvent DMF only were added. Experimental conditions and results are reported in Table 4.

**Table 4:**

| Exp. No. | c(PyOxim) [g/l (mM)] | V (DIPEA) [ml] | V(peptide sol. added) [ml] | c(PyOxim) in solution added [g/l (mM)] | Cyclic monomer [%] | Dimer [%] | Other side products [%] |
|---|---|---|---|---|---|---|---|
| 9 | 62.95 (119.4) | 0.625 | 10 | 0(0) | 93.80 | 0.85 | 5.34 |
| 10 | 31.48 (59.7) | 0.625 | 10 | 0(0) | 92.11 | 0.84 | 7.03 |

The results from Table 4 confirm that an increase in reaction volume is deleterious to the yield and purity of the desired cyclic monomer.

### Example 5: Retention of coupling reagent by nanofiltration

2.5 l of PyOxim solution in DMF at a concentration of 1 g/l (1.9 mM) were loaded into a stirred reaction vessel, which also functioned as the feed tank of a nanofiltration system. Said system comprised a retentate loop with pressure sensors and a pressure control valve, a recirculation pump, and a nanofiltration unit with a flat-sheet polymeric membrane (filtration area of 54 cm²). Commercially available membranes made from modified polyimide with a molecular weight cut-off of 150 g/mol or 200 g/mol were used during this experiment.

In order to measure the retention of PyOxim, the liquid was circulated within the nanofiltration system with a flow rate of 1.2 l/min. The pressure in the retentate loop was set at 20 bar. Samples of permeate and retentate were taken at various time points and the concentration of PyOxim was determined by means of UV-Vis spectroscopy. The results are presented in Table 5.

**Table 5:**

| Time [min] | 50 | 120 | 180 | 250 | 330 | 390 | 450 | 540 |
|---|---|---|---|---|---|---|---|---|
| PyOxim retention Duramem^{®} 150 [%] | 97.3 | 98.7 | 99.0 | 99.8 | 99.8 | 99.8 | 99.7 | 99.6 |
| PyOxim retention Duramem^{®} 200 [%] | 90.4 | 95.1 | 95.1 | 98.8 | 99.1 | 99.2 | 98.9 | 99.0 |

As is illustrated in Table 5, the apparatus according to the present invention allows retaining the coupling reagent within the reaction vessel.

### Example 6: Cyclisation of peptide with increasing reaction volume

Raw peptide 1 will be dissolved in 1.25 l of DMF at a concentration of 80 g/l (58.7 mM). In a separate container, 2.5 l of a 40.3 g/l (76.4 mM) solution of coupling reagent (PyOxim) will be prepared. The first solution will be transferred to a first feed tank, whereas the other one will be placed directly in the stirred reaction vessel. The first feed tank will be connected to the stirred reaction vessel. The reaction will be started by gradually adding the solution from the first feed tank into the reaction vessel. The liquid present in the reaction vessel will be circulated within the nanofiltration system without applying any pressure. Samples of reaction mixture will be taken at regular time points and then analyzed by analytical RP-HPLC. After the completion of the reaction, the nanofiltration system will be depressurized and drained to recover the retentate, to quantify final purity and yield.

### Example 7: Nanofiltration assisted peptide cyclization with constant reaction volume

Raw peptide 1 will be dissolved in 1.25 l of DMF at a concentration of 80 g/l (58.7 M). In a separate container, 2.5 l of a 40.3 g/l (76.4 mM) solution of coupling reagent (PyOxim) will be prepared. The first solution will be transferred to a first feed tank, whereas the other one will be placed directly in the stirred reaction vessel. The first feed tank will be connected to the stirred reaction vessel. The reaction will be started by gradually adding the solution from the first feed tank to the reaction vessel. The volumetric flow of peptide solution will be adjusted so that it is equal to the volumetric flow rate of permeate. The liquid present in the reaction vessel will be circulated within the nanofiltration system at 20 bar before starting the reaction and during the whole time of the reagent addition from the storage vessel. Samples of permeate and retentate will be taken at regular time points and then analyzed by analytical RP-HPLC. After the completion of the reaction, the nanofiltration system will be depressurized and drained to recover the retentate.

### Example 8: Nanofiltration assisted peptide cyclization with constant volume and constant addition of coupling reagent

Raw peptide 1 will be dissolved in 0.75 l of DMF at a concentration of 133.33 g/l (97.7 mM). In a separate container, 0.5 l of a 100.7 g/l (191.0 mM) solution of coupling reagent (PyOxim) will be prepared. These solutions will be transferred to a first and second feed tank, respectively. The reaction vessel is filled with 2.5 l of 40.3 g/l (76.4 mM) coupling reagent solution. The feed tanks will be connected via locally separated inlets to the stirred reaction vessel. The reaction will be started by gradually adding the solution from the feed tanks to the reaction vessel. The liquid present in the reaction vessel will be circulated within the nanofiltration system at 20 bar before starting the reaction and during the whole time of the reagent addition from the storage vessel. Samples of permeate and retentate will be taken at regular time points and then analyzed by analytical RP-HPLC. After the completion of the reaction, the nanofiltration system will be depressurized and drained to recover the retentate.

### Example 9: Simulation of concentration profiles during the cyclisation reaction

The theoretical concentration profiles of reactants and products during the cyclisation reaction were simulated numerically using Matlab^{®} software. Molecular weights of reactants and products as well as general operating conditions and system parameters used for the simulations are listed in Table 6.

**Table 6:**

| Description | Value | Unit |
|---|---|---|
| Molecular weight linear peptide | 1364 | g/mol |
| Molecular weight coupling reagent | 527 | g/mol |
| Molecular weight cyclic peptide | 1346 | g/mol |
| Permeance of the membrane | 3.0 | l/(m² bar h) |
| Filtration area | 0.0108 | m² |
| Initial volume of coupling reagent solution in reaction vessel | 2.0 | l |
| Cyclisation reaction rate constant | 10⁴ | l/(mol h) |
| Rejection of linear and cyclic peptides | 100 | % |

The simulation was carried out for four different cases:
9.1) Solution of coupling reagent is loaded into the reaction vessel and peptide solution is fed from the feed tank. During the whole process no pressure is applied. The simulated setup corresponds to the experimental setup as in Example 6.
9.2) Solution of coupling reagent is loaded to the reaction vessel and peptide solution is fed from the feed tank. During the whole process a constant pressure is applied. The simulated setup corresponds to the experimental setup as in Example 7.
9.3) Solution of coupling reagent is loaded to the reaction vessel. Both peptide and coupling reagent solutions are fed from separate feed tanks. During the whole process a constant pressure is applied. The simulated setup corresponds to the experimental setup as in Example 8.
9.4) Solution of coupling reagent is loaded to the reaction vessel, with half the concentration of case 9.3. Both peptide and coupling reagent solutions are fed from the feed tanks. During the whole process a constant pressure is applied. The simulated setup corresponds to the experimental setup as in Example 8.

The simulated concentration profiles for case studies 9.1 to 9.4 are shown in Figure 2. Clearly, in case study 9.1 the concentration profile of the cyclic peptide is approaching a plateau due to intrinsic dilution caused by continuous addition of peptide solution. Concentration of the cyclic product during reaction is possible only to a certain extent, as the overall process efficiency is limited by the self-dilution effect. On the other hand, when nanofiltration is applied (case studies 9.2-9.4), the concentration of cyclic peptide increases linearly and can be theoretically increased up to the solubility limit of the cyclic peptide. In case study 9.2 the concentration of the coupling reagent decreases monotonically, due to its consumption by the cyclisation reaction. This phenomenon is compensated by the addition of coupling reagent solution in case studies 9.3 and 9.4, where the concentration of coupling reagent remains constant. The optimal starting concentration of coupling reagent in the reaction vessel must be found for the specific case study and may be different from the optimal starting concentration for the classical batch process as in case 9.1 (case 9.4 vs 9.3). All case studies simulate instantaneous consumption of linear peptide, which determines the very low concentration of linear peptide in the reaction mixture throughout the whole reaction time. The specific parameters used for each case study are listed in Table 7.

**Table 7:**

| Parameter | Case study 9.1 | Case study 9.2 | Case study 9.3 | Case study 9.4 |
|---|---|---|---|---|
| Peptide concentration in storage vessel [mmol/l] | 9.5 | 9.5 | 13.6 | 13.6 |
| Coupling reagent concentration in storage vessel [mmol/l] | 0.0 | 0.0 | 31.8 | 31.8 |
| Initial coupling reagent concentration in reaction vessel [mmol/l] | 9.5 | 9.5 | 9.5 | 4.8 |
| Initial volume of peptide solution in storage vessel [l] | 1.0 | 1.0 | 0.7 | 0.7 |
| Initial volume of coupling reagent solution in storage vessel [l] | 0.0 | 0.0 | 0.3 | 0.3 |
| Volumetric flow of peptide solution [l/h] | 0.648 | 0.648 | 0.454 | 0.454 |
| Volumetric flow of coupling reagent solution [l/h] | 0.0 | 0.0 | 0.195 | 0.195 |
| Differential pressure across the membrane [bar] | 0.0 | 20.0 | 20.0 | 20.0 |

### Example 10: Nanofiltration assisted peptide cyclization

### General procedure

Raw peptide 1 was dissolved in DMF at variable concentration (see Tables 8-10, line 1) and 3 eq of DIPEA were added. In a separate container, a solution of coupling reagent (PyOxim) in DMF at variable concentration (see Tables 8-10, line 2) was prepared. These solutions were transferred to a first and second feed tank, respectively. The reaction vessel was filled with a solution of coupling reagent in DMF at variable concentration (see Tables 8-10 line 3). The feed tanks were connected via locally separated inlets to the stirred reaction vessel. The reaction was started by gradually adding the solution from the feed tanks to the reaction vessel. The liquid present in the reaction vessel was circulated within the nanofiltration system at variable pressure (see Tables 8-10 line 11) before starting the reaction and during the whole time of the reagent addition from the feed tank. The pressure in the system was adjusted so that the volumetric flow of the permeate was equal to the sum of volumetric flows of feed solutions 1 and 2. Samples of permeate and retentate were taken at regular time points and then analyzed by analytical RP-HPLC as described above. The sum of the areas of all significant peaks, which were not identified as cyclic monomer and had a retention time higher than the cyclic monomer, was used to quantify the amount of side products. After the completion of the reaction, i.e. at the end of reagent addition, the nanofiltration system was depressurized and drained to recover the retentate. The reaction performance was assessed based on the relative amount of peptidic side products in the retentate (Tables 8-10, line 15).

### Example 10.1: Effect of constant reaction volume and continuous addition of coupling reagent.

In the method according to the present invention, the reaction conditions may be controlled, e.g., by keeping the reaction volume and consequently the concentration of the linear peptide in the reaction mixture constant and by continuously adding coupling reagent in order to balance its consumption by the cyclisation reaction and loss across the membrane. The effect of these measures on the amount of side products formed is shown in Table 8. If no membrane filtration was performed and the peptide solution was merely added into an excess of coupling reagent inside the reactor (experiment T1), 5.48% of side products were observed. Using membrane filtration to keep the volume of the reaction mixture constant over time (-experiment T2) resulted in a reduction to 4.40% of side products, and using membrane filtration plus addition of coupling reagent (experiment T3A) gave a further reduction to 3.90% of side products. Hence an improvement of approx. 30% is observed when membrane filtration is applied and coupling reagent is topped-up so as to keep its concentration within the reaction vessel constant.

**Table 8**

| **No.** | **Parameter** | **Experiment No.** | | |
|---|---|---|---|---|
| | | **T1** | **T2** | **T3A** |
| 1 | Peptide concentration in feed tank 1 [mmol/l] | 91.64 | 91.64 | 91.64 |
| 2 | Coupling reagent concentration in feed tank 2 [mmol/l] | 0 | 0 | 278.54 |
| 3 | Initial coupling reagent concentration in reaction vessel [mmol/l] | 69.62 | 69.62 | 69.62 |
| 4 | Initial volume of peptide solution in feed tank 1 [l] | 0.014 | 0.014 | 0.014 |
| 5 | Initial volume of coupling reagent solution in feed tank 2 [l] | 0.005 | 0.005 | 0.005 |
| 6 | Initial volume of coupling reagent solution in reaction vessel [l] | 0.050 | 0.050 | 0.050 |
| 7 | Total permeate volume [l] | 0 | 0.019 | 0.025 |
| 8 | Volumetric flow of peptide solution [l/h] | 0.027 | 0.027 | 0.027 |
| 9 | Volumetric flow of coupling reagent solution [l/h] | 0.009 | 0.009 | 0.009 |
| 10 | Volumetric flow of permeate stream [l/h] | 0 | 0.036 | 0.036 |
| 11 | Differential pressure across the membrane [bar] | 0 | 40 | 44 |
| 12 | MWCO of Duramem^{®} membrane [g/mol] | 150 | 150 | 150 |
| 13 | Membrane area [m²] | 0 | 0.0066 | 0.0066 |
| 14 | Operating time [min] | 31 | 31 | 31 |
| **15** | **Side products [%]** | **5.48** | **4.40** | **3.90** |

### Example 10.2: Effect of diafiltration during the reaction

Performing membrane filtration during the course of the reaction to keep the reaction volume constant may give an inline diafiltration effect:this may result in the removal of (usually small) molecular species, which pass through the membrane filtration unit more easily than the reactants and the desired products. Such species may be introduced together with the reagents or may be formed during the reaction. To assess whether the removal of such molecular species from the reaction mixture influences product purity, variations in the total volume of liquid flux through the reaction vessel during the reaction time and in the molecular weight cut-off (MWCO) of the membrane filtration unit were tested.

In experiment T3E vs. T3D, the total volume of permeate formed was 5 times higher (see Table 9, experiments T3D and T3E, line 7), but the liquid volume inside the reaction vessel was kept constant. This was achieved by using a more dilute solution of coupling reagent in feed tank 2, i.e. a larger volume of coupling reagent solution was added to the reaction vessel in order to deliver the same amount of coupling reagent. To counter this influx, the volumetric flow of permeate out of the reaction vessel was increased by a factor of five (see Table 9, experiments T3D and T3E, line 10). As a result of this increased inline diafiltration, the percentage of side products formed dropped from 5.39% to 2.47%, which is a reduction by 54%.

In experiment T3B vs. T3J, two membrane filtration units with different MWCOs (200 vs. 500) were compared. As the membrane used in T3J was found to have a rejection of 90% for PyOxim, the loss of coupling reagent needed to be compensated by adding more coupling reagent in order to keep its concentration within the reaction vessel constant. This is reflected in the larger concentration of coupling reagent in feed tank 2 (see Table 9, experiments T3B and T3J, line 2). For practical reasons, the whole system (reaction vessel volume, amount of peptide cyclized, membrane area etc.) was scaled down by a factor of 2 in experiment T3J vs T3B, but no other parameters were changed. It was found that using a membrane with a higher MWCO resulted in a reduction of side products from 2.71% to 2.16%.

**Table 9:**

| **No.** | **Parameter** | **Experiment No.** | | | |
|---|---|---|---|---|---|
| | | **T3D** | **T3E** | **T3B** | **T3J** |
| 1 | Peptide concentration in feed tank 1 [mmol/l] | 18.33 | 18.33 | 3.67 | 3.67 |
| 2 | Coupling reagent concentration in feed tank 2 [mmol/l] | 55.69 | 3.28 | 11.13 | 39.00 |
| 3 | Initial coupling reagent concentration in reaction vessel [mmol/l] | 69.62 | 69.62 | 69.62 | 69.62 |
| 4 | Initial volume of peptide solution in feed tank 1 [l] | 0.067 | 0.067 | 0.336 | 0.168 |
| 5 | Initial volume of coupling reagent solution in feed tank 2 [l] | 0.023 | 0.381 | 0.112 | 0.056 |
| 6 | Initial volume of coupling reagent solution in reaction vessel [l] | 0.050 | 0.050 | 0.050 | 0.025 |
| 7 | Total permeate volume [l] | 0.090 | 0.448 | 0.448 | 0.224 |
| 8 | Volumetric flow of peptide solution [l/h] | 0.013 | 0.013 | 0.067 | 0.034 |
| 9 | Volumetric flow of coupling reagent solution [l/h] | 0.005 | 0.076 | 0.022 | 0.011 |
| 10 | Volumetric flow of permeate stream [l/h] | 0.018 | 0.090 | 0.090 | 0.045 |
| 11 | Differential pressure across the membrane [bar] | 9 | 23 | 38 | 7 |
| 12 | MWCO of Duramem^{®} membrane [g/mol] | 200 | 200 | 200 | 500 |
| 13 | Membrane area [m²] | 0.0066 | 0.0066 | 0.0066 | 0.0033 |
| 14 | Operating time [min] | 300 | 300 | 300 | 300 |
| **15** | **Side products [%]** | **5.39** | **2.47** | **2.71** | **2.16** |

### Example 10.3: Effect of concentration of coupling reagent

The present setup allows keeping the concentration of coupling reagent constant over the entire reaction time. It was investigated how the concentration of the coupling reagent inside the reaction vessel influences product purity. For practical reasons, the whole system (reaction vessel volume, amount of peptide cyclized, membrane area etc.) was scaled down by a factor of 2 in experiment T3F and T3G vs T3B, but no parameters other than the initial concentration of coupling reagent were changed. It was found that decreasing the excess of coupling reagent in the reaction vessel (2 vs. 1.25 vs 0.5 molar equivalents of the total amount of peptide added) while keeping the same linear peptide and coupling reagent concentrations in the feed solutions (Table 10, lines 1-3) and the same rates of reagent addition causes a decrease in product purity (Table 10, line 15). In other words: a higher excess of the coupling reagent (second compound) over the linear peptide (first compound) corresponds to a lower amount of by-products.

**Table 10:**

| **No.** | **Parameter** | **Experiment No.** | | |
|---|---|---|---|---|
| | | **T3B** | **T3G** | **T3F** |
| 1 | Peptide concentration in feed tank 1 [mmol/l] | 3.67 | 3.67 | 3.67 |
| 2 | Coupling reagent concentration in feed tank 2 [mmol/l] | 11.13 | 11.13 | 11.13 |
| 3 | Initial coupling reagent concentration in reaction vessel [mmol/l] | 69.62 | 43.52 | 17.40 |
| 4 | Initial volume of peptide solution in feed tank 1 [l] | 0.336 | 0.168 | 0.168 |
| 5 | Initial volume of coupling reagent solution in feed tank 2 [l] | 0.112 | 0.056 | 0.056 |
| 6 | Initial volume of coupling reagent solution in reaction vessel [l] | 0.050 | 0.025 | 0.025 |
| 7 | Total permeate volume [l] | 0.448 | 0.224 | 0.224 |
| 8 | Volumetric flow of peptide solution [l/h] | 0.067 | 0.034 | 0.034 |
| 9 | Volumetric flow of coupling reagent solution [l/h] | 0.022 | 0.011 | 0.011 |
| 10 | Volumetric flow of permeate stream [l/h] | 0.090 | 0.045 | 0.045 |
| 11 | Differential pressure across the membrane [bar] | 38 | 38 | 33 |
| 12 | MWCO of Duramem^{®} membrane [g/mol] | 200 | 200 | 200 |
| 13 | Membrane area [m²] | 0.0066 | 0.0033 | 0.0033 |
| 14 | Operating time [min] | 300 | 300 | 300 |
| **15** | **Side products [%]** | **2.71** | **4.77** | **5.29** |

### Example 10.4: Effect of solvent recycling (permeate solution from previous experiments as a solvent)

### Comparative example T3C:

The same reaction conditions as in T3B were applied (see Table 9). The only difference was the solvent used to prepare the coupling reagent solution in reaction vessel. In T3C the permeate collected during T3B was used (without further purification), whereas in T3B fresh DMF was used for the same purpose.

### Comparative example T3H:

The same reaction conditions as in T3B were applied (see Table 9). The only difference was the solvent used to prepare the peptide solution in feed tank 1. The solvent was prepared as follows: the nanofiltration system was cleaned after performing T3F and then the permeate collected during T3F was transferred to the clean retentate loop. This permeate solution was subjected to concentration using the same membrane as in T3B and T3H (MWCO of 200 g/mol). During concentration, the volume of the solution in the retentate loop was reduced as much as possible, and subsequently discarded. The retentate loop was then cleaned. The permeate was collected in a separate vessel and then transferred back to the clean retentate loop. The concentration was repeated 2 more times. The final permeate solution from the 3^{rd} concentration was mixed with fresh DMF (62.5vol% permeate, 37.5vol% DMF) and used to dissolve the peptide used in feed tank 1.

A quantitative comparison of overall reaction performance between tests mentioned above is presented in Table 11.

**Table 11:**

| **Experiment No.** | **T3B** | **T3C** | **T3H** |
|---|---|---|---|
| **Side products [%]** | **2.71** | **5.63** | **3.73** |

When recycled solvent was used instead of fresh solvent, the level of impurities roughly doubled (5.63% vs. 2.71%). The results suggest that recycling used solvent leads to reintroduction of molecular species, which may enhance the formation of peptidic side products. Such species could not be satisfactorily removed even by repeated membrane filtration, as is shown in experiment T3H.

## Claims

1. A method for reacting a first compound and a second compound, thereby forming a third compound, the method comprising the following steps:
a) Providing a first solution S1 comprising the first compound and a second solution S2 comprising the second compound;
b) Providing, inside a reaction vessel, a solution S3 comprising the second compound in a liquid reaction medium, which comprises an organic solvent; and
c) Concomitantly adding the solutions S1 and S2 over an extended period of time into the solution S3 while mixing the content of the reaction vessel, thereby forming a reaction mixture comprising the third compound;
wherein:
d) The second compound is in excess over the first compound inside the reaction vessel during step c); and
e) During step c), the content of the reaction vessel is subjected to membrane filtration so as to essentially retain the first compound, the second compound, and the third compound inside the reaction vessel.

2. The method according to claim 1, wherein the excess of the second compound over the first compound inside the reaction vessel during step c) is essentially constant over time.

3. The method according to any one of claims 1 to 2, further wherein a liquid, which is miscible with the reaction mixture, is added into the reaction vessel while subjecting the content of the reaction vessel to membrane filtration.

4. The method according to any one of claims 1 to 3, further wherein the volume of liquid inside the reaction vessel is kept essentially constant over the duration of step c).

5. The method according to claims 3 or 4, wherein the content of the reaction vessel is subjected to diafiltration after completion of step c).

6. The method according to any one of claims 1 to 5, wherein the third compound is metastable.

7. The method according to claim 6, wherein the metastable third compound undergoes a spontaneous cyclization reaction, thereby forming a cyclic fourth compound.

8. The method according to any one of claims 1 to 7, further comprising a step of incubating the third compound with a fifth compound, thereby forming a sixth compound.

9. The method according to any one of claims 1 to 8, wherein at least one of said first and second compounds comprises a carboxyl group or a hydroxyl group.

10. The method according to any one of claims 1 to 9, wherein the equivalent concentration of the second compound inside the reaction vessel is higher than the equivalent concentration of the first compound inside the reaction vessel by a factor of at least 1.5.

11. The method according to any one of claims 1 to 10, wherein the content of the reaction vessel is conducted to a cross-flow membrane filtration unit and the retentate from said cross-flow membrane filtration unit is conducted back into the reaction vessel.

12. The method according to any one of claims 1 to 11, wherein the rejection rate of the membrane employed is at least 90% for each of compound 1, compound 2, and compound 3.

13. The method according to any one of claims 7 to 12, wherein the fourth compound is a cyclic peptide.

14. The method according to any one of claims 1 to 13, wherein the first compound is a peptide and the second compound is a carboxyl group activating reagent.

15. The method according to any one of claims 1 to 14, further wherein a cyclic peptide or peptide carrier conjugate produced is subjected to a step of purification or isolation selected from the group consisting of:
- diafiltration,
- a concentration step,
- crystallization,
- lyophilization,
- precipitation,
- dialysis,
- chromatography, and
- reversed phase high performance liquid chromatography.

## Patentansprüche

1. Verfahren zum Reagieren einer ersten Verbindung und einer zweiten Verbindung, dabei Bilden einer dritten Verbindung, wobei das Verfahren die folgenden Schritte umfasst:
a) Bereitstellen einer ersten Lösung S1 umfassend die erste Verbindung und einer zweiten Lösung S2 umfassend die zweite Verbindung;
b) Bereitstellen, innerhalb eines Reaktionsgefäßes, eine Lösung S3 umfassend die zweite Verbindung in einem flüssigen Reaktionsmedium, das ein organisches Lösungsmittel umfasst;
c) Gleichzeitiges Hinzufügen der Lösungen S1 und S2 über eine längeren Zeitraum in die Lösung S3 währenddessen Mischen des Inhalts des Reaktionsgefäßes, dabei Bilden einer Reaktionsmischung umfassend die dritte Verbindung;
wobei
d) Die zweite Verbindung im Überschuss über die erste Verbindung im Reaktionsgefäß während Schritt c) vorliegt; und
e) Während Schritt c) der Inhalt des Reaktionsgefäßes Membranfiltration unterzogen wird, um im Wesentlichen die erste Verbindung, die zweite Verbindung und die dritte Verbindung innerhalb des Reaktionsgefäßes zurückzubehalten.

2. Verfahren gemäß Anspruch 1, wobei der Überschuss an der zweiten Verbindung über die erste Verbindung innerhalb des Reaktionsgefäßes während Schritt c) im Wesentlichen konstant über die Zeit ist.

3. Verfahren gemäß einem beliebigen der Ansprüche 1 bis 2, wobei außerdem eine Flüssigkeit, welche mit der Reaktionsmischung mischbar ist, in das Reaktionsgefäß hinzugefügt wird, während der Inhalt des Reaktionsgefäßes Membranfiltration unterzogen wird.

4. Verfahren gemäß einem beliebigen der Ansprüche 1 bis 3, wobei außerdem das Volumen der Flüssigkeit innerhalb des Reaktionsgefäßes während der Dauer des Schritts c) im Wesentlichen konstant gehalten wird.

5. Verfahren gemäß Ansprüchen 3 oder 4, wobei der Inhalt des Reaktionsgefäßes nach Abschluss von Schritt c) Diafiltration unterzogen wird.

6. Verfahren gemäß einem beliebigen der Ansprüche 1 bis 5, wobei die dritte Verbindung metastabil ist.

7. Verfahren gemäß Anspruch 6, wobei die metastabile dritte Verbindung eine spontane Zyklisierungsreaktion durchläuft, dabei eine zyklische vierte Verbindung bildend.

8. Verfahren gemäß einem beliebigen der Ansprüche 1 bis 7, außerdem umfassend einen Schritt des Inkubierens der dritten Verbindung mit einer fünften Verbindung, dabei eine sechste Verbindung bildend.

9. Verfahren gemäß einem beliebigen der Ansprüche 1 bis 8, wobei mindestens eine der ersten und zweiten Verbindungen eine Carboxylgruppe oder eine Hydroxylgruppe umfasst.

10. Verfahren gemäß einem beliebigen der Ansprüche 1 bis 9, wobei die Äquivalenzkonzentration der zweiten Verbindung innerhalb des Reaktionsgefäßes um einen Faktor von mindestens 1,5 höher ist als die Äquivalenzkonzentration der ersten Verbindung innerhalb des Reaktionsgefäßes.

11. Verfahren gemäß einem beliebigen der Ansprüche 1 bis 10, wobei der Inhalt des Reaktionsgefäßes auf Querstrom-Membran-Filtrationseinheit geleitet wird und das Retentat aus der Querstrom-Membran-Filtrationseinheit in das Reaktionsgefäß zurückgeleitet wird.

12. Verfahren gemäß einem beliebigen der Ansprüche 1 bis 11, wobei die Rückweisungsrate der eingesetzten Membran mindestens 90% für jede der Verbindung 1, Verbindung 2 und Verbindung 3 beträgt.

13. Verfahren gemäß einem beliebigen der Ansprüche 7 bis 12, wobei die vierte Verbindung ein zyklisches Peptid ist.

14. Verfahren gemäß einem beliebigen der Ansprüche 1 bis 13, wobei die erste Verbindung ein Peptid ist und die zweite Verbindung ein Carboxylgruppenaktivierendes Reagenz ist.

15. Verfahren gemäß einem beliebigen der Ansprüche 1 bis 14, wobei außerdem ein zyklisches Peptid oder gebildetes Peptid-Träger-Konjugat einem Schritt der Reinigung oder Isolation unterzogen wird, ausgewählt aus der Gruppe besehend aus:
- Diafiltration,
- einem Konzentrierungsschritt,
- Kristallisation,
- Lyophilisation,
- Fällung,
- Dialyse,
- Chromatographie, und
- Umkehrphasen-Hochleistungsflüssigkeitschromatographie.

## Revendications

1. Procédé de réaction d'un premier composé et d'un deuxième composé, formant ainsi un troisième composé, le procédé comprenant les étapes suivantes :
a) Fourniture d'une première solution S1 comprenant le premier composé et d'une deuxième solution S2 comprenant le deuxième composé ;
b) Fourniture, à l'intérieur d'une cuve de réaction, d'une solution S3 comprenant le deuxième composé dans un milieu de réaction liquide, qui comprend un solvant organique ; et
c) L'ajout concomitant des solutions S1 et S2 sur une période prolongée dans la solution S3 tout en mélangeant le contenu de la cuve de réaction, formant ainsi un mélange réactionnel comprenant le troisième composé ;
dans lequel :
d) Le deuxième composé est en excès par rapport au premier composé à l'intérieur de la cuve de réaction pendant l'étape c) ; et
e) Pendant l'étape c), le contenu de la cuve de réaction est soumis à filtration sur membrane de sorte à retenir essentiellement le premier composé, le deuxième composé et le troisième composé à l'intérieur de la cuve de réaction.

2. Procédé selon la revendication 1, dans lequel l'excès du deuxième composé par rapport au premier composé à l'intérieur de la cuve de réaction pendant l'étape c) est essentiellement constant au cours du temps.

3. Procédé selon l'une quelconque des revendications 1 à 2, en outre dans lequel un liquide, qui est miscible au mélange réactionnel, est ajouté dans la cuve de réaction tout en soumettant le contenu de la cuve de réaction à filtration sur membrane.

4. Procédé selon l'une quelconque des revendications 1 à 3, en outre dans lequel le volume de liquide à l'intérieur de la cuve de réaction est maintenu à un niveau essentiellement constant sur la durée de l'étape c).

5. Procédé selon les revendications 3 ou 4, dans lequel le contenu de la cuve de réaction est soumis à diafiltration après l'achèvement de l'étape c).

6. Procédé selon l'une quelconque des revendications 1 à 5, dans lequel le troisième composé est métastable.

7. Procédé selon la revendication 6, dans lequel le troisième composé métastable subit une réaction de cyclisation spontanée, formant ainsi un quatrième composé cyclique.

8. Procédé selon l'une quelconque des revendications 1 à 7, comprenant en outre une étape d'incubation du troisième composé avec un cinquième composé, formant ainsi un sixième composé.

9. Procédé selon l'une quelconque des revendications 1 à 8, dans lequel au moins l'un parmi lesdits premier et deuxième composés comprend un groupement carboxyle ou un groupement hydroxyle.

10. Procédé selon l'une quelconque des revendications 1 à 9, dans lequel la concentration équivalente du deuxième composé à l'intérieur de la cuve de réaction est supérieure à la concentration équivalente du premier composé à l'intérieur de la cuve de réaction par un facteur d'au moins 1,5.

11. Procédé selon l'une quelconque des revendications 1 à 10, dans lequel le contenu de la cuve de réaction est dirigé vers une unité de filtration sur membrane à flux transversal et le rétentat de ladite unité de filtration sur membrane à flux transversal est redirigé vers la cuve de réaction.

12. Procédé selon l'une quelconque des revendications 1 à 11, dans lequel le taux de rétention de la membrane employée est d'au moins 90 % pour chacun parmi le composé 1, le composé 2 et le composé 3.

13. Procédé selon l'une quelconque des revendications 7 à 12, dans lequel le quatrième composé est un peptide cyclique.

14. Procédé selon l'une quelconque des revendications 1 à 13, dans lequel le premier composé est un peptide et le deuxième composé est un réactif activateur de groupement carboxyle.

15. Procédé selon l'une quelconque des revendications 1 à 14, en outre dans lequel un peptide cyclique ou un conjugué peptide-support produit est soumis à une étape de purification ou d'isolement choisie dans le groupe constitué par :
- la diafiltration,
- une étape de concentration,
- la cristallisation,
- la lyophilisation,
- la précipitation,
- la dialyse,
- la chromatographie, et
- la chromatographie liquide haute performance en phase inverse.
